(19) Europäisches Patentamt
European Patent Office
Office européen des brevets

(11) EP 1 124 426 B1

(12) EUROPEAN PATENT SPECIFICATION

(45) Date of publication and mention of the grant of the patent:
**22.01.2003 Bulletin 2003/04**

(21) Application number: **00955863.6**

(22) Date of filing: **23.08.2000**

(51) Int Cl.$^7$: **A01N 63/00**, A01N 63/02

(86) International application number:
**PCT/US00/23156**

(87) International publication number:
**WO 01/013731 (01.03.2001 Gazette 2001/09)**

# (54) METHODS OF CONTROLLING CUTWORM PESTS

VERFAHREN ZUR BEKÄMPFUNG VON NACHTFALTERLARVEN

PROCEDES DESTINES A LA LUTTE CONTRE LE VER-GRIS

(84) Designated Contracting States:
**AT BE CH CY DE DK ES FI FR GB GR IE IT LI LU MC NL PT SE**

(30) Priority: **23.08.1999 US 150319 P**

(43) Date of publication of application:
**22.08.2001 Bulletin 2001/34**

(73) Proprietor: **Mycogen Corporation**
**San Diego, CA 92121 (US)**

(72) Inventors:
- **STOCKHOFF, Brian, A.**
  **San Diego, CA 92131 (US)**
- **CONLAN, Christopher**
  **San Diego, CA 92126 (US)**

(74) Representative: **Perry, Robert Edward**
**GILL JENNINGS & EVERY**
**Broadgate House**
**7 Eldon Street**
**London EC2M 7LH (GB)**

(56) References cited:
**WO-A-91/16434** **WO-A-94/05771**
**WO-A-98/18932** **WO-A-99/24581**

- **J.MÜLLER-COHN ET AL.: "Spodoptera littoralis (Lepidoptera: Noctuidae) Resistance to CryIC and Cross-Resistance to Other Bacillus thuringiensis Crystal Toxins" JOURNAL OF ECONOMIC ENTOMOLOGY., vol. 89, no. 4, August 1996 (1996-08), pages 791-796, XP002154101 ENTOMOLOGICAL SOCIETY OF AMERICA. COLLEGE PARK, MARYLAND., US ISSN: 0022-0493**
- **DATABASE BIOSIS [Online] BIOSCIENCES INFORMATION SERVICE, PHILADELPHIA, PA, US; H.S.SALAMA ET AL.: "The use of Bacilus thuringiensis to control two lepidopterous insect pests (Agrotis ypsilon and and Spodoptera littoralis)" retrieved from EPOQUE, accession no. prev199598279392 XP002154102 & AZEIGER FÜR SCHÄDLINGSKUNDE PFLANZENSCHUTZ UMWELTSCHUTZ, vol. 68, 1995, pages 15-17,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Mycogen Corporation

<120> Methods of Controlling Cutworm Pests

<130> MA-731XC1

<140>

<141>

<160> 8

<170> PatentIn Ver. 2.0

<210> 1

<211> 3444

<212> DNA

<213> Artificial Sequence

<220>

<223> Synthetic B.t. toxin gene

<4001> 1

```
atggagaaca acatacagaa tcagtgcgtc ccctacaact gcctcaacaa tcctgaagta 60
gagattctca acgaagagag gtcgactggc agattgccgt tagacatctc cctgtccctt 120
acacgtttcc tgttgtctga gtttgttcca ggtgtgggag ttgcgtttgg cctcttcgac 180
ctcatctggg gcttcatcac tccatctgat tggagcctct ttcttctcca gattgaacag 240
ttgattgaac aaaggattga gaccttggaa aggaatcggg ccatcactac ccttcgtggc 300
ttagcagaca gctatgagat ctacattgaa gcactaagag agtgggaagc caatcctaac 360
aatgcccaac tgagagaaga tgtgcgtata cgctttgcta acacagatga tgctttgatc 420
acagccatca acaacttcac ccttaccagc ttcgagatcc ctcttctctc ggtctatgtt 480
caagctgcta acctgcactt gtcactactg cgcgacgctg tgtcgtttgg gcaaggttgg 540
ggactggaca tagctactgt caacaatcac tacaacagac tcatcaatct gattcatcga 600
tacacgaaac attgtttgga tacctacaat cagggattgg agaacctgag aggtactaac 660
actcgccaat gggccaggtt caatcagttc aggagagacc ttacacttac tgtgttagac 720
atagttgctc tctttccgaa ctacgatgtt cgtacctatc cgattcaaac gtcatcccaa 780
cttacaaggg agatctacac cagttcagtc attgaagact ctccagtttc tgcgaacata 840
cccaatggtt tcaacagggc tgagtttgga gtcagaccac cccatctcat ggacttcatg 900
aactctttgt ttgtgactgc agagactgtt agatcccaaa ctgtgtgggg aggacactta 960
gttagctcac gcaacacggc tggcaatcgt atcaactttc ctagttacgg ggtcttcaat 1020
cccgggggcg ccatctggat tgcagatgaa gatccacgtc ctttctatcg gaccttgtca 1080
gatcctgtct tcgtccgagg aggctttggc aatcctcact atgtactcgg tcttagggga 1140
gtggcctttc aacaaactgg tacgaatcac acccgcacat tcaggaactc cgggaccatt 1200
gactctctag atgagatacc acctcaagac aacagcggcg caccttggaa tgactactcc 1260
catgtgctga atcatgttac ctttgtgcgc tggccaggtg agatctcagg ttccgactca 1320
tggagagcac caatgttctc ttggacgcat cgtagcgcta cccccacaaa caccattgat 1380
ccagagagaa tcactcagat tcccttggtg aaggcacaca cacttcagtc aggaactaca 1440
gttgtaagag ggccggggtt cacgggagga gacattcttc gacgcactag tggaggacca 1500
ttcgcgtaca ccattgtcaa catcaatggg caacttcccc aaaggtatcg tgccaggata 1560
cgctatgcct ctactaccaa tctaagaatc tacgttacgg ttgcaggtga acggatcttt 1620
gctggtcagt tcaacaagac aatggatacc ggtgatccac ttacattcca atctttctcc 1680
tacgccacta tcaacaccgc gttcaccttt ccaatgagcc agagcagttt cacagtaggt 1740
```

```
gctgatacct tcagttcagg caacgaagtg tacattgaca ggtttgagtt gattccagtt 1800
actgccacac tcgaggcaga gtctgacttg gaaagagcac agaaggcggt gaatgctctg 1860
ttcacttcgt ccaatcagat tgggctcaag acagatgtga ctgactatca catcgatcgc 1920
gtttccaacc ttgttgagtg cctctctgat gagttctgtt tggatgagaa gaaggagttg 1980
tccgagaagg tcaaacatgc taagcgactt agtgatgagc ggaacttgct tcaagatccc 2040
aactttcgcg ggatcaacag gcaactagat cgtggatgga ggggaagtac ggacatcacc 2100
attcaaggag gtgatgatgt gttcaaggag aactatgtta cgctcttggg tacctttgat 2160
gagtgctatc caacatacct gtaccagaag atagatgaat cgaaactcaa agcctacaca 2220
agataccagt tgagaggtta catcgaggac agtcaagacc ttgagatcta cctcatcaga 2280
tacaacgcca aacatgagac agtcaatgtg cctgggacgg gttcactctg gccactttca 2340
gccccaagtc ccatcggcaa gtgtgcccat cactcacacc acttctcctt ggacatagac 2400
gttggctgta ccgacctgaa cgaagacctc ggtgtgtggg tgatcttcaa gatcaagact 2460
caagatggcc atgccaggct aggcaatctg gagtttctag aagagaaacc acttgttgga 2520
gaagccctcg ctagagtgaa gagggctgag aagaagtgga gggacaagag agagaagttg 2580
gaatgggaaa caaacattgt gtacaaagaa gccaaagaaa gcgttgacgc tctgtttgtg 2640
aactctcagt atgataggct ccaagctgat accaacatag ctatgattca tgctgcagac 2700
aaacgcgttc atagcattcg ggaagcttac cttcctgaac ttagcgtgat tccgggtgtc 2760
aatgctgcta tctttgaaga gttagaaggg cgcatcttca ctgcattctc cttgtatgat 2820
gcgaggaatg tcatcaagaa tggtgacttc aacaatggcc tatcctgctg gaatgtgaaa 2880
gggcacgtag atgtagaaga acagaacaat caccgctctg tccttgttgt tcctgagtgg 2940
gaagcagaag tttcacaaga agttcgtgtc tgtcctggtc gtggctacat tcttcgtgtt 3000
accgcgtaca aagaaggata cggagaaggt tgcgtcacca tacacgagat tgagaacaac 3060
accgacgagc tgaagttcag caactgcgtc gaggaggaag tctacccaaa caacaccgta 3120
acttgcaatg actacactgc gactcaagag gagtatgagg gtacttacac ttctcgcaat 3180
cgaggatacg atggagccta tgagagcaac tcttctgtac ccgctgacta tgcatcagcc 3240
tatgaggaga aggcttacac cgatggacgt agggacaatc cttgcgaatc taacagaggc 3300
tatggggact acacaccgtt accagccggc tatgtcacca aagagttaga gtactttcca 3360
gaaaccgaca aggtttggat tgagattgga gaaacggaag gaacattcat tgttgatagc 3420
gtggagttac ttctgatgga ggaa                                       3444
```

<210> 2

<211> 1148

<212> PRT

<213> Artificial Sequence

<220>

<223> Toxin encoded by synthetic B.T. gene

<400> 2

```
Met Glu Asn Asn Ile Gln Asn Gln Cys Val Pro Tyr Asn Cys Leu Asn
 1               5                  10                  15

Asn Pro Glu Val Glu Ile Leu Asn Glu Glu Arg Ser Thr Gly Arg Leu
            20                  25                  30

Pro Leu Asp Ile Ser Leu Ser Leu Thr Arg Phe Leu Leu Ser Glu Phe
        35                  40                  45

Val Pro Gly Val Gly Val Ala Phe Gly Leu Phe Asp Leu Ile Trp Gly
    50                  55                  60

Phe Ile Thr Pro Ser Asp Trp Ser Leu Phe Leu Leu Gln Ile Glu Gln
65                  70                  75                  80
```

```
Leu Ile Glu Gln Arg Ile Glu Thr Leu Glu Arg Asn Arg Ala Ile Thr
                85                  90                  95

Thr Leu Arg Gly Leu Ala Asp Ser Tyr Glu Ile Tyr Ile Glu Ala Leu
            100                 105                 110

Arg Glu Trp Glu Ala Asn Pro Asn Asn Ala Gln Leu Arg Glu Asp Val
        115                 120                 125

Arg Ile Arg Phe Ala Asn Thr Asp Asp Ala Leu Ile Thr Ala Ile Asn
    130                 135                 140

Asn Phe Thr Leu Thr Ser Phe Glu Ile Pro Leu Leu Ser Val Tyr Val
145                 150                 155                 160

Gln Ala Ala Asn Leu His Leu Ser Leu Leu Arg Asp Ala Val Ser Phe
                165                 170                 175

Gly Gln Gly Trp Gly Leu Asp Ile Ala Thr Val Asn Asn His Tyr Asn
            180                 185                 190

Arg Leu Ile Asn Leu Ile His Arg Tyr Thr Lys His Cys Leu Asp Thr
        195                 200                 205

Tyr Asn Gln Gly Leu Glu Asn Leu Arg Gly Thr Asn Thr Arg Gln Trp
    210                 215                 220

Ala Arg Phe Asn Gln Phe Arg Arg Asp Leu Thr Leu Thr Val Leu Asp
225                 230                 235                 240

Ile Val Ala Leu Phe Pro Asn Tyr Asp Val Arg Thr Tyr Pro Ile Gln
                245                 250                 255

Thr Ser Ser Gln Leu Thr Arg Glu Ile Tyr Thr Ser Ser Val Ile Glu
            260                 265                 270

Asp Ser Pro Val Ser Ala Asn Ile Pro Asn Gly Phe Asn Arg Ala Glu
        275                 280                 285

Phe Gly Val Arg Pro Pro His Leu Met Asp Phe Met Asn Ser Leu Phe
    290                 295                 300

Val Thr Ala Glu Thr Val Arg Ser Gln Thr Val Trp Gly Gly His Leu
305                 310                 315                 320

Val Ser Ser Arg Asn Thr Ala Gly Asn Arg Ile Asn Phe Pro Ser Tyr
                325                 330                 335

Gly Val Phe Asn Pro Gly Gly Ala Ile Trp Ile Ala Asp Glu Asp Pro
            340                 345                 350

Arg Pro Phe Tyr Arg Thr Leu Ser Asp Pro Val Phe Val Arg Gly Gly
        355                 360                 365
```

Phe Gly Asn Pro His Tyr Val Leu Gly Leu Arg Gly Val Ala Phe Gln
    370                 375                 380

Gln Thr Gly Thr Asn His Thr Arg Thr Phe Arg Asn Ser Gly Thr Ile
385                 390                 395                 400

Asp Ser Leu Asp Glu Ile Pro Pro Gln Asp Asn Ser Gly Ala Pro Trp
                405                 410                 415

Asn Asp Tyr Ser His Val Leu Asn His Val Thr Phe Val Arg Trp Pro
            420                 425                 430

Gly Glu Ile Ser Gly Ser Asp Ser Trp Arg Ala Pro Met Phe Ser Trp
        435                 440                 445

Thr His Arg Ser Ala Thr Pro Thr Asn Thr Ile Asp Pro Glu Arg Ile
    450                 455                 460

Thr Gln Ile Pro Leu Val Lys Ala His Thr Leu Gln Ser Gly Thr Thr
465                 470                 475                 480

Val Val Arg Gly Pro Gly Phe Thr Gly Gly Asp Ile Leu Arg Arg Thr
                485                 490                 495

Ser Gly Gly Pro Phe Ala Tyr Thr Ile Val Asn Ile Asn Gly Gln Leu
            500                 505                 510

Pro Gln Arg Tyr Arg Ala Arg Ile Arg Tyr Ala Ser Thr Thr Asn Leu
        515                 520                 525

Arg Ile Tyr Val Thr Val Ala Gly Glu Arg Ile Phe Ala Gly Gln Phe
    530                 535                 540

Asn Lys Thr Met Asp Thr Gly Asp Pro Leu Thr Phe Gln Ser Phe Ser
545                 550                 555                 560

Tyr Ala Thr Ile Asn Thr Ala Phe Thr Phe Pro Met Ser Gln Ser Ser
                565                 570                 575

Phe Thr Val Gly Ala Asp Thr Phe Ser Ser Gly Asn Glu Val Tyr Ile
            580                 585                 590

Asp Arg Phe Glu Leu Ile Pro Val Thr Ala Thr Leu Glu Ala Glu Ser
        595                 600                 605

Asp Leu Glu Arg Ala Gln Lys Ala Val Asn Ala Leu Phe Thr Ser Ser
    610                 615                 620

Asn Gln Ile Gly Leu Lys Thr Asp Val Thr Asp Tyr His Ile Asp Arg
625                 630                 635                 640

Val Ser Asn Leu Val Glu Cys Leu Ser Asp Glu Phe Cys Leu Asp Glu
                645                 650                 655

Lys Lys Glu Leu Ser Glu Lys Val Lys His Ala Lys Arg Leu Ser Asp
660 665 670

Glu Arg Asn Leu Leu Gln Asp Pro Asn Phe Arg Gly Ile Asn Arg Gln
675 680 685

Leu Asp Arg Gly Trp Arg Gly Ser Thr Asp Ile Thr Ile Gln Gly Gly
690 695 700

Asp Asp Val Phe Lys Glu Asn Tyr Val Thr Leu Leu Gly Thr Phe Asp
705 710 715 720

Glu Cys Tyr Pro Thr Tyr Leu Tyr Gln Lys Ile Asp Glu Ser Lys Leu
725 730 735

Lys Ala Tyr Thr Arg Tyr Gln Leu Arg Gly Tyr Ile Glu Asp Ser Gln
740 745 750

Asp Leu Glu Ile Tyr Leu Ile Arg Tyr Asn Ala Lys His Glu Thr Val
755 760 765

Asn Val Pro Gly Thr Gly Ser Leu Trp Pro Leu Ser Ala Pro Ser Pro
770 775 780

Ile Gly Lys Cys Ala His His Ser His His Phe Ser Leu Asp Ile Asp
785 790 795 800

Val Gly Cys Thr Asp Leu Asn Glu Asp Leu Gly Val Trp Val Ile Phe
805 810 815

Lys Ile Lys Thr Gln Asp Gly His Ala Arg Leu Gly Asn Leu Glu Phe
820 825 830

Leu Glu Glu Lys Pro Leu Val Gly Glu Ala Leu Ala Arg Val Lys Arg
835 840 845

Ala Glu Lys Lys Trp Arg Asp Lys Arg Glu Lys Leu Glu Trp Glu Thr
850 855 860

Asn Ile Val Tyr Lys Glu Ala Lys Glu Ser Val Asp Ala Leu Phe Val
865 870 875 880

Asn Ser Gln Tyr Asp Arg Leu Gln Ala Asp Thr Asn Ile Ala Met Ile
885 890 895

His Ala Ala Asp Lys Arg Val His Ser Ile Arg Glu Ala Tyr Leu Pro
900 905 910

Glu Leu Ser Val Ile Pro Gly Val Asn Ala Ala Ile Phe Glu Glu Leu
915 920 925

Glu Gly Arg Ile Phe Thr Ala Phe Ser Leu Tyr Asp Ala Arg Asn Val
930 935 940

Ile Lys Asn Gly Asp Phe Asn Asn Gly Leu Ser Cys Trp Asn Val Lys
945 950 955 960

Gly His Val Asp Val Glu Glu Gln Asn Asn His Arg Ser Val Leu Val
965 970 975

Val Pro Glu Trp Glu Ala Glu Val Ser Gln Glu Val Arg Val Cys Pro
980 985 990

Gly Arg Gly Tyr Ile Leu Arg Val Thr Ala Tyr Lys Glu Gly Tyr Gly
995 1000 1005

Glu Gly Cys Val Thr Ile His Glu Ile Glu Asn Asn Thr Asp Glu Leu
1010 1015 1020

Lys Phe Ser Asn Cys Val Glu Glu Glu Val Tyr Pro Asn Asn Thr Val
1025 1030 1035 1040

Thr Cys Asn Asp Tyr Thr Ala Thr Gln Glu Glu Tyr Glu Gly Thr Tyr
1045 1050 1055

Thr Ser Arg Asn Arg Gly Tyr Asp Gly Ala Tyr Glu Ser Asn Ser Ser
1060 1065 1070

Val Pro Ala Asp Tyr Ala Ser Ala Tyr Glu Glu Lys Ala Tyr Thr Asp
1075 1080 1085

Gly Arg Arg Asp Asn Pro Cys Glu Ser Asn Arg Gly Tyr Gly Asp Tyr
1090 1095 1100

Thr Pro Leu Pro Ala Gly Tyr Val Thr Lys Glu Leu Glu Tyr Phe Pro
1105 1110 1115 1120

Glu Thr Asp Lys Val Trp Ile Glu Ile Gly Glu Thr Glu Gly Thr Phe
1125 1130 1135

Ile Val Asp Ser Val Glu Leu Leu Leu Met Glu Glu
1140 1145

<210> 3

<211> 1815

<212> DNA

<213> Artificial Sequence

<220>

<223> Synthetic B.t. toxin gene

<400> 3

```
atggagaaca acatacagaa tcagtgcgtc ccctacaact gcctcaacaa tcctgaagta 60
gagattctca acgaagagag gtcgactggc agattgccgt tagacatctc cctgtccctt 120
acacgtttcc tgttgtctga gtttgttcca ggtgtgggag ttgcgtttgg cctcttcgac 180
ctcatctggg gcttcatcac tccatctgat tggagcctct ttcttctcca gattgaacag 240
ttgattgaac aaaggattga gaccttggaa aggaatcggg ccatcactac ccttcgtggc 300
ttagcagaca gctatgagat ctacattgaa gcactaagag agtgggaagc caatcctaac 360

aatgcccaac tgagagaaga tgtgcgtata cgctttgcta acacagatga tgctttgatc 420
acagccatca acaacttcac ccttaccagc ttcgagatcc ctcttctctc ggtctatgtt 480
caagctgcta acctgcactt gtcactactg cgcgacgctg tgtcgtttgg gcaaggttgg 540
ggactggaca tagctactgt caacaatcac tacaacagac tcatcaatct gattcatcga 600
tacacgaaac attgtttgga tacctacaat cagggattgg agaacctgag aggtactaac 660
actcgccaat gggccaggtt caatcagttc aggagagacc ttacacttac tgtgttagac 720
atagttgctc tctttccgaa ctacgatgtt cgtacctatc cgattcaaac gtcatcccaa 780
cttacaaggg agatctacac cagttcagtc attgaagact ctccagtttc tgcgaacata 840
cccaatggtt tcaacagggc tgagtttgga gtcagaccac cccatctcat ggacttcatg 900
aactctttgt ttgtgactgc agagactgtt agatcccaaa ctgtgtgggg aggacactta 960
gttagctcac gcaacacggc tggcaatcgt atcaactttc ctagttacgg ggtcttcaat 1020
cccgggggcg ccatctggat tgcagatgaa gatccacgtc ctttctatcg gaccttgtca 1080
gatcctgtct tcgtccgagg aggctttggc aatcctcact atgtactcgg tcttagggga 1140
gtggcctttc aacaaactgg tacgaatcac acccgcacat tcaggaactc cggaccatt 1200
gactctctag atgagatacc acctcaagac aacagcggcg caccttggaa tgactactcc 1260
catgtgctga atcatgttac ctttgtgcgc tggccaggtg agatctcagg ttccgactca 1320
tggagagcac caatgttctc ttggacgcat cgtagcgcta cccccacaaa caccattgat 1380
ccagagagaa tcactcagat tcccttggtg aaggcacaca cacttcagtc aggaactaca 1440
gttgtaagag ggccggggtt cacgggagga gacattcttc gacgcactag tggaggacca 1500
ttcgcgtaca ccattgtcaa catcaatggg caacttcccc aaaggtatcg tgccaggata 1560
cgctatgcct ctactaccaa tctaagaatc tacgttacgg ttgcaggtga acggatcttt 1620
gctggtcagt tcaacaagac aatggatacc ggtgatccac ttacattcca atctttctcc 1680
tacgccacta tcaacaccgc gttcaccttt ccaatgagcc agagcagttt cacagtaggt 1740
gctgatacct tcagttcagg caacgaagtg tacattgaca ggtttgagtt gattccagtt 1800
actgccacac tcgag                                                 1815
```

<210> 4

<211> 605

<212> PRT

<213> Artificial Sequence

<220>

<223> Toxin encoded by synthetic B.t. gene

<400> 4

Met Glu Asn Asn Ile Gln Asn Gln Cys Val Pro Tyr Asn Cys Leu Asn
1 5 10 15

Asn Pro Glu Val Glu Ile Leu Asn Glu Glu Arg Ser Thr Gly Arg Leu
20 25 30

Pro Leu Asp Ile Ser Leu Ser Leu Thr Arg Phe Leu Leu Ser Glu Phe
35 40 45

Val Pro Gly Val Gly Val Ala Phe Gly Leu Phe Asp Leu Ile Trp Gly
50 55 60

Phe Ile Thr Pro Ser Asp Trp Ser Leu Phe Leu Leu Gln Ile Glu Gln
65 70 75 80

Leu Ile Glu Gln Arg Ile Glu Thr Leu Glu Arg Asn Arg Ala Ile Thr
85 90 95

Thr Leu Arg Gly Leu Ala Asp Ser Tyr Glu Ile Tyr Ile Glu Ala Leu
100 105 110

Arg Glu Trp Glu Ala Asn Pro Asn Asn Ala Gln Leu Arg Glu Asp Val
115 120 125

Arg Ile Arg Phe Ala Asn Thr Asp Asp Ala Leu Ile Thr Ala Ile Asn
130 135 140

Asn Phe Thr Leu Thr Ser Phe Glu Ile Pro Leu Leu Ser Val Tyr Val
145 150 155 160

Gln Ala Ala Asn Leu His Leu Ser Leu Leu Arg Asp Ala Val Ser Phe
165 170 175

Gly Gln Gly Trp Gly Leu Asp Ile Ala Thr Val Asn Asn His Tyr Asn
180 185 190

Arg Leu Ile Asn Leu Ile His Arg Tyr Thr Lys His Cys Leu Asp Thr
195 200 205

Tyr Asn Gln Gly Leu Glu Asn Leu Arg Gly Thr Asn Thr Arg Gln Trp
210 215 220

Ala Arg Phe Asn Gln Phe Arg Arg Asp Leu Thr Leu Thr Val Leu Asp
225 230 235 240

Ile Val Ala Leu Phe Pro Asn Tyr Asp Val Arg Thr Tyr Pro Ile Gln
245 250 255

Thr Ser Ser Gln Leu Thr Arg Glu Ile Tyr Thr Ser Ser Val Ile Glu
260 265 270

Asp Ser Pro Val Ser Ala Asn Ile Pro Asn Gly Phe Asn Arg Ala Glu
275 280 285

Phe Gly Val Arg Pro Pro His Leu Met Asp Phe Met Asn Ser Leu Phe
290 295 300

Val Thr Ala Glu Thr Val Arg Ser Gln Thr Val Trp Gly Gly His Leu
305 310 315 320

Val Ser Ser Arg Asn Thr Ala Gly Asn Arg Ile Asn Phe Pro Ser Tyr
325 330 335

Gly Val Phe Asn Pro Gly Gly Ala Ile Trp Ile Ala Asp Glu Asp Pro
340 345 350

Arg Pro Phe Tyr Arg Thr Leu Ser Asp Pro Val Phe Val Arg Gly Gly
355 360 365

Phe Gly Asn Pro His Tyr Val Leu Gly Leu Arg Gly Val Ala Phe Gln
370 375 380

Gln Thr Gly Thr Asn His Thr Arg Thr Phe Arg Asn Ser Gly Thr Ile
385 390 395 400

```
Asp Ser Leu Asp Glu Ile Pro Pro Gln Asp Asn Ser Gly Ala Pro Trp
                405                 410                 415

Asn Asp Tyr Ser His Val Leu Asn His Val Thr Phe Val Arg Trp Pro
            420                 425                 430

Gly Glu Ile Ser Gly Ser Asp Ser Trp Arg Ala Pro Met Phe Ser Trp
        435                 440                 445

Thr His Arg Ser Ala Thr Pro Thr Asn Thr Ile Asp Pro Glu Arg Ile
    450                 455                 460

Thr Gln Ile Pro Leu Val Lys Ala His Thr Leu Gln Ser Gly Thr Thr
465                 470                 475                 480

Val Val Arg Gly Pro Gly Phe Thr Gly Gly Asp Ile Leu Arg Arg Thr
                485                 490                 495

Ser Gly Gly Pro Phe Ala Tyr Thr Ile Val Asn Ile Asn Gly Gln Leu
            500                 505                 510

Pro Gln Arg Tyr Arg Ala Arg Ile Arg Tyr Ala Ser Thr Thr Asn Leu
        515                 520                 525

Arg Ile Tyr Val Thr Val Ala Gly Glu Arg Ile Phe Ala Gly Gln Phe
    530                 535                 540

Asn Lys Thr Met Asp Thr Gly Asp Pro Leu Thr Phe Gln Ser Phe Ser
545                 550                 555                 560

Tyr Ala Thr Ile Asn Thr Ala Phe Thr Phe Pro Met Ser Gln Ser Ser
                565                 570                 575

Phe Thr Val Gly Ala Asp Thr Phe Ser Ser Gly Asn Glu Val Tyr Ile
            580                 585                 590

Asp Arg Phe Glu Leu Ile Pro Val Thr Ala Thr Leu Glu
        595                 600                 605
```

<210> 5

<211> 3522

<212> DNA

<213> Bacillus thuringiensis

<400> 5

atggagaata atattcaaaa tcaatgcgta ccttacaatt gtttaaataa tcctgaagta 60
gaaatattaa atgaagaaag aagtactggc agattaccgt tagatatatc cttatcgctt 120
acacgtttcc ttttgagtga atttgttcca ggtgtgggag ttgcgtttgg attatttgat 180
ttaatatggg gttttataac tccttctgat tggagcttat ttcttttaca gattgaacaa 240
ttgattgagc aaagaataga aacattggaa aggaaccggg caattactac attacgaggg 300
ttagcagata gctatgaaat ttatattgaa gcactaagag agtgggaagc aaatcctaat 360
aatgcacaat taaggaaaga tgtgcgtatt cgatttgcta atacagacga cgctttaata 420
acagcaataa ataattttac acttacaagt tttgaaatcc ctcttttatc ggtctatgtt 480
caagcggcga atttacattt atcactatta agagacgctg tatcgtttgg gcagggttgg 540

```
ggactggata tagctactgt taataatcat tataatagat taataaatct tattcataga 600
tatacgaaac attgtttgga cacatacaat caaggattag aaaacttaag aggtactaat 660
actcgacaat gggcaagatt caatcagttt aggagagatt taacacttac tgtattagat 720
atcgttgctc tttttccgaa ctacgatgtt agaacatatc caattcaaac gtcatcccaa 780
ttaacaaggg aaatttatac aagttcagta attgaggatt ctccagtttc tgctaatata 840
cctaatggtt ttaatagggc ggaatttgga gttagaccgc cccatcttat ggactttatg 900
aattctttgt ttgtaactgc agagactgtt agaagtcaaa ctgtgtgggg aggacactta 960
gttagttcac gaaatacggc tggtaaccgt ataaatttcc ctagttacgg ggtcttcaat 1020
cctggtggcg ccatttggat tgcagatgag gatccacgtc ctttttatcg gacattatca 1080
gatcctgttt ttgtccgagg aggatttggg aatcctcatt atgtactggg gcttagggga 1140
gtagcatttc aacaaactgg tacgaaccac acccgaacat ttagaaatag tgggaccata 1200
gattctctag atgaaatccc acctcaggat aatagtgggg caccttggaa tgattatagt 1260
catgtattaa atcatgttac atttgtacga tggccaggtg agatttcagg aagtgattca 1320
tggagagctc caatgttttc ttggacgcac cgtagtgcaa cccctacaaa tacaattgat 1380
ccggagagga ttactcaaat accattggta aaagcacata cacttcagtc aggtactact 1440
gttgtaagag ggcccgggtt tacgggagga gatattcttc gacgaacaag tggaggacca 1500
tttgcttata ctattgttaa tataaatggg caattacccc aaaggtatcg tgcaagaata 1560
cgctatgcct ctactacaaa tctaagaatt tacgtaacgg ttgcaggtga acggattttt 1620
gctggtcaat ttaacaaaac aatggatacc ggtgacccat taacattcca atcttttagt 1680
tacgcaacta ttaatacagc ttttacattc ccaatgagcc agagtagttt cacagtaggt 1740
gctgatactt ttagttcagg gaatgaagtt tatatagaca gatttgaatt gattccagtt 1800
actgcaacat ttgaagcaga atatgattta gaaagagcac aaaaggcggt gaatgcgctg 1860
tttacttcta taaaccaaat agggataaaa acagatgtga cggattatca tattgatcaa 1920
gtatccaatt tagtggattg tttatcagat gaattttgtc tggatgaaaa gcgagaattg 1980
tccgagaaag tcaaacatgc gaagcgactc agtgatgagc ggaatttact tcaagatcca 2040
aacttcaaag gcatcaatag gcaactagac cgtggttgga gaggaagtac ggatattacc 2100
atccaaagag gagatgacgt attcaaagaa aattatgtca cactaccagg tacctttgat 2160
gagtgctatc caacgtattt atatcaaaaa atagatgagt cgaaattaaa accctatact 2220
cgttatcaat taagagggta tatcgaggat agtcaagact tagaaatcta tttgatccgc 2280
tataatgcaa aacacgaaac agtaaatgtg ctaggtacgg gttctttatg gccgctttca 2340
gtccaaagtc caatcagaaa gtgtggagaa ccgaatcgat gcgcgccaca ccttgaatgg 2400
aatcctgatc tagattgttc ctgcagagac ggggaaaaat gtgcacatca ttcgcatcat 2460
ttctccttgg acattgatgt tggatgtaca gacttaaatg aggacttaga tgtatgggtg 2520
atattcaaga ttaagacgca agatggccat gcaagactag gaaatctaga gtttctcgaa 2580
gagaaaccat tagtcgggga agcactagct cgtgtgaaaa gagcagagaa aaaatggaga 2640
gataaacgtg aaaaattgga attggaaaca aatattgttt ataaagaggc aaaagaatct 2700
gtagatgctt tatttgtaaa ctctcaatat gatcaattac aagcggatac gaatattgcc 2760
atgattcatg cggcagataa acgtgttcat agaattcggg aagcgtatct tccagagtta 2820
tctgtgattc cgggtgtaaa tgtagacatt ttcgaagaat taaaagggcg tattttcact 2880
gcattcttcc tatatgatgc gagaaatgtc attaaaaacg gtgatttcaa taatggctta 2940
tcatgctgga acgtgaaagg gcatgtagat gtagaagaac aaaacaacca ccgttcggtc 3000
cttgttgttc cggaatggga agcagaagtg tcacaagaag ttcgtgtctg tccgggtcgt 3060
ggctatatcc ttcgtgtcac agcgtacaag gagggatatg gagaaggttg cgtaaccatt 3120
catgagatcg agaacaatac agacgaactg aagtttagca actgcgtaga agaggaagtc 3180
tatccaaaca acacggtaac gtgtaatgat tatactgcaa atcaagaaga atacgggggt 3240
gcgtacactt cccgtaatcg tggatatgac gaaacttatg gaagcaattc ttctgtacca 3300
gctgattatg cgtcagtcta tgaagaaaaa tcgtatacag atggacgaag agacaatcct 3360
tgtgaatcta acagaggata tggggattac acaccactac cagctggcta tgtgacaaaa 3420
gaattagagt acttcccaga aaccgataag gtatggattg agatcggaga aacggaagga 3480
acattcatcg tggacagcgt ggaattactc cttatggagg aa                    3522
```

<210> 6

<211> 1174

<212> PRT

<213> Bacillus thuringiensis

<400> 6

Met Glu Asn Asn Ile Gln Asn Gln Cys Val Pro Tyr Asn Cys Leu Asn
1 5 10 15

Asn Pro Glu Val Glu Ile Leu Asn Glu Glu Arg Ser Thr Gly Arg Leu
20 25 30

Pro Leu Asp Ile Ser Leu Ser Leu Thr Arg Phe Leu Leu Ser Glu Phe
35 40 45

Val Pro Gly Val Gly Val Ala Phe Gly Leu Phe Asp Leu Ile Trp Gly
50 55 60

Phe Ile Thr Pro Ser Asp Trp Ser Leu Phe Leu Leu Gln Ile Glu Gln
65 70 75 80

Leu Ile Glu Gln Arg Ile Glu Thr Leu Glu Arg Asn Arg Ala Ile Thr
85 90 95

Thr Leu Arg Gly Leu Ala Asp Ser Tyr Glu Ile Tyr Ile Glu Ala Leu
100 105 110

Arg Glu Trp Glu Ala Asn Pro Asn Asn Ala Gln Leu Arg Glu Asp Val
115 120 125

Arg Ile Arg Phe Ala Asn Thr Asp Asp Ala Leu Ile Thr Ala Ile Asn
130 135 140

Asn Phe Thr Leu Thr Ser Phe Glu Ile Pro Leu Leu Ser Val Tyr Val
145 150 155 160

Gln Ala Ala Asn Leu His Leu Ser Leu Leu Arg Asp Ala Val Ser Phe
165 170 175

Gly Gln Gly Trp Gly Leu Asp Ile Ala Thr Val Asn Asn His Tyr Asn
180 185 190

Arg Leu Ile Asn Leu Ile His Arg Tyr Thr Lys His Cys Leu Asp Thr
195 200 205

Tyr Asn Gln Gly Leu Glu Asn Leu Arg Gly Thr Asn Thr Arg Gln Trp
210 215 220

Ala Arg Phe Asn Gln Phe Arg Arg Asp Leu Thr Leu Thr Val Leu Asp
225 230 235 240

Ile Val Ala Leu Phe Pro Asn Tyr Asp Val Arg Thr Tyr Pro Ile Gln
245 250 255

Thr Ser Ser Gln Leu Thr Arg Glu Ile Tyr Thr Ser Ser Val Ile Glu
260 265 270

Asp Ser Pro Val Ser Ala Asn Ile Pro Asn Gly Phe Asn Arg Ala Glu
275 280 285

Phe Gly Val Arg Pro Pro His Leu Met Asp Phe Met Asn Ser Leu Phe
    290                     295                     300

Val Thr Ala Glu Thr Val Arg Ser Gln Thr Val Trp Gly Gly His Leu
305                     310                     315                     320

Val Ser Ser Arg Asn Thr Ala Gly Asn Arg Ile Asn Phe Pro Ser Tyr
                325                     330                     335

Gly Val Phe Asn Pro Gly Gly Ala Ile Trp Ile Ala Asp Glu Asp Pro
            340                     345                     350

Arg Pro Phe Tyr Arg Thr Leu Ser Asp Pro Val Phe Val Arg Gly Gly
        355                     360                     365

Phe Gly Asn Pro His Tyr Val Leu Gly Leu Arg Gly Val Ala Phe Gln
    370                     375                     380

Gln Thr Gly Thr Asn His Thr Arg Thr Phe Arg Asn Ser Gly Thr Ile
385                     390                     395                     400

Asp Ser Leu Asp Glu Ile Pro Pro Gln Asp Asn Ser Gly Ala Pro Trp
                405                     410                     415

Asn Asp Tyr Ser His Val Leu Asn His Val Thr Phe Val Arg Trp Pro
            420                     425                     430

Gly Glu Ile Ser Gly Ser Asp Ser Trp Arg Ala Pro Met Phe Ser Trp
        435                     440                     445

Thr His Arg Ser Ala Thr Pro Thr Asn Thr Ile Asp Pro Glu Arg Ile
    450                     455                     460

Thr Gln Ile Pro Leu Val Lys Ala His Thr Leu Gln Ser Gly Thr Thr
465                     470                     475                     480

Val Val Arg Gly Pro Gly Phe Thr Gly Gly Asp Ile Leu Arg Arg Thr
                485                     490                     495

Ser Gly Gly Pro Phe Ala Tyr Thr Ile Val Asn Ile Asn Gly Gln Leu
            500                     505                     510

Pro Gln Arg Tyr Arg Ala Arg Ile Arg Tyr Ala Ser Thr Thr Asn Leu
        515                     520                     525

Arg Ile Tyr Val Thr Val Ala Gly Glu Arg Ile Phe Ala Gly Gln Phe
    530                     535                     540

Asn Lys Thr Met Asp Thr Gly Asp Pro Leu Thr Phe Gln Ser Phe Ser
545                     550                     555                     560

Tyr Ala Thr Ile Asn Thr Ala Phe Thr Phe Pro Met Ser Gln Ser Ser
                565                     570                     575

Phe Thr Val Gly Ala Asp Thr Phe Ser Ser Gly Asn Glu Val Tyr Ile
580 585 590

Asp Arg Phe Glu Leu Ile Pro Val Thr Ala Thr Phe Glu Ala Glu Tyr
595 600 605

Asp Leu Glu Arg Ala Gln Lys Ala Val Asn Ala Leu Phe Thr Ser Ile
610 615 620

Asn Gln Ile Gly Ile Lys Thr Asp Val Thr Asp Tyr His Ile Asp Gln
625 630 635 640

Val Ser Asn Leu Val Asp Cys Leu Ser Asp Glu Phe Cys Leu Asp Glu
645 650 655

Lys Arg Glu Leu Ser Glu Lys Val Lys His Ala Lys Arg Leu Ser Asp
660 665 670

Glu Arg Asn Leu Leu Gln Asp Pro Asn Phe Lys Gly Ile Asn Arg Gln
675 680 685

Leu Asp Arg Gly Trp Arg Gly Ser Thr Asp Ile Thr Ile Gln Arg Gly
690 695 700

Asp Asp Val Phe Lys Glu Asn Tyr Val Thr Leu Pro Gly Thr Phe Asp
705 710 715 720

Glu Cys Tyr Pro Thr Tyr Leu Tyr Gln Lys Ile Asp Glu Ser Lys Leu
725 730 735

Lys Pro Tyr Thr Arg Tyr Gln Leu Arg Gly Tyr Ile Glu Asp Ser Gln
740 745 750

Asp Leu Glu Ile Tyr Leu Ile Arg Tyr Asn Ala Lys His Glu Thr Val
755 760 765

Asn Val Leu Gly Thr Gly Ser Leu Trp Pro Leu Ser Val Gln Ser Pro
770 775 780

Ile Arg Lys Cys Gly Glu Pro Asn Arg Cys Ala Pro His Leu Glu Trp
785 790 795 800

Asn Pro Asp Leu Asp Cys Ser Cys Arg Asp Gly Glu Lys Cys Ala His
805 810 815

His Ser His His Phe Ser Leu Asp Ile Asp Val Gly Cys Thr Asp Leu
820 825 830

Asn Glu Asp Leu Asp Val Trp Val Ile Phe Lys Ile Lys Thr Gln Asp
835 840 845

Gly His Ala Arg Leu Gly Asn Leu Glu Phe Leu Glu Glu Lys Pro Leu
850 855 860

Val Gly Glu Ala Leu Ala Arg Val Lys Arg Ala Glu Lys Lys Trp Arg
865 870 875 880

Asp Lys Arg Glu Lys Leu Glu Leu Glu Thr Asn Ile Val Tyr Lys Glu
885 890 895

Ala Lys Glu Ser Val Asp Ala Leu Phe Val Asn Ser Gln Tyr Asp Gln
900 905 910

Leu Gln Ala Asp Thr Asn Ile Ala Met Ile His Ala Ala Asp Lys Arg
915 920 925

Val His Arg Ile Arg Glu Ala Tyr Leu Pro Glu Leu Ser Val Ile Pro
930 935 940

Gly Val Asn Val Asp Ile Phe Glu Glu Leu Lys Gly Arg Ile Phe Thr
945 950 955 960

Ala Phe Phe Leu Tyr Asp Ala Arg Asn Val Ile Lys Asn Gly Asp Phe
965 970 975

Asn Asn Gly Leu Ser Cys Trp Asn Val Lys Gly His Val Asp Val Glu
980 985 990

Glu Gln Asn Asn His Arg Ser Val Leu Val Val Pro Glu Trp Glu Ala
995 1000 1005

Glu Val Ser Gln Glu Val Arg Val Cys Pro Gly Arg Gly Tyr Ile Leu
1010 1015 1020

Arg Val Thr Ala Tyr Lys Glu Gly Tyr Gly Glu Gly Cys Val Thr Ile
1025 1030 1035 1040

His Glu Ile Glu Asn Asn Thr Asp Glu Leu Lys Phe Ser Asn Cys Val
1045 1050 1055

Glu Glu Glu Val Tyr Pro Asn Asn Thr Val Thr Cys Asn Asp Tyr Thr
1060 1065 1070

Ala Asn Gln Glu Glu Tyr Gly Gly Ala Tyr Thr Ser Arg Asn Arg Gly
1075 1080 1085

Tyr Asp Glu Thr Tyr Gly Ser Asn Ser Ser Val Pro Ala Asp Tyr Ala
1090 1095 1100

Ser Val Tyr Glu Glu Lys Ser Tyr Thr Asp Gly Arg Arg Asp Asn Pro
1105 1110 1115 1120

Cys Glu Ser Asn Arg Gly Tyr Gly Asp Tyr Thr Pro Leu Pro Ala Gly
1125 1130 1135

Tyr Val Thr Lys Glu Leu Glu Tyr Phe Pro Glu Thr Asp Lys Val Trp
1140 1145 1150

```
Ile Glu Ile Gly Glu Thr Glu Gly Thr Phe Ile Val Asp Ser Val Glu
        1155                1160                1165

Leu Leu Leu Met Glu Glu
    1170
```

<210> 7

<211> 1815

<212> DNA

<213> Artificial Sequence

<220>

<223> Synthetic B.t. toxin gene

<400> 7

```
atggagaaca acatacagaa tcagtgtgtc ccctacaact gcctcaacaa tcctgaagta 60
gagattctca acgaagaaag gtcgactggc agattgccgt tagacatctc cctgtccctt 120
acacgattcc tgttgtctga gttcgttcct ggtgtgggtg ttgcgtttgg cctcttcgat 180
ctcatctggg ggttcatcac tccatctgat tggagcctct ttcttctaca gattgaacag 240
ttgattgaac aaaggattga gaccttagaa aggaatcggg ccatcactac acttcgtggg 300
ttagcagaca gctatgagat ctacattgaa gcactaagag agtgggaagc caatcctaac 360
aatgcacaac tgagagaaga tgtgcgcata cgctttgcta acacagatga tgctttgatc 420
acagccatca acaacttcac acttaccagc ttcgagattc ctcttctctc ggtctatgtt 480
caagctgcta accttcactt gtcactactg agggatgctg tgtcgtttgg ccaaggttgg 540
ggactggaca tagctactgt caacaatcac tacaacagac tcatcaatct gattcatcga 600
tacacgaaac attgtttgga tacctacaat cagggattgg agaatctgag aggtactaac 660
actcgtcaat gggctaggtt caatcagttc aggagagacc ttacacttac tgtgttagac 720
atagttgctc tctttccgaa ctatgatgtt cgtacctatc cgattcaaac gtcatcccaa 780
cttacaaggg agatctacac cagttcagtc attgaagact ctccagtttc tgcgaacata 840
ccgaatggtt tcaacagggc tgagtttgga gtcagacctc cccatctcat ggacttcatg 900
aactctttgt ttgtgactgc agaaactgtt agatcgcaaa ctgtgtgggg aggacactta 960
gttagctcaa ggaacacggc tggcaatcgt atcaactttc ctagttacgg ggtcttcaat 1020
cccggggtg ccatctggat tgcagatgaa gatccacgtc ctttctatcg gaccttgtca 1080
gatcctgtct tcgttcgagg aggctttggc aatcctcact atgtactagg tcttagggga 1140
gtggcctttc aacaaactgg tacgaatcac acacgcacat tcaggaactc cgggaccatt 1200
gactctctag atgagatacc acctcaagac aacagcggcg caccttggaa tgactactcg 1260
catgtgctga atcatgttac ctttgtgcgc tggccaggtg agatctctgg ttccgactca 1320
tggagagcac ctatgttctc ttggacgcat cgtagcgcta cacctacaaa caccattgat 1380
ccagaaagaa tcactcagat tcccttggtg aaggcacaca cacttcagtc aggaactaca 1440
gttgtaagag ggccggggtt cacgggagga gacattcttc gaaggactag tggaggacca 1500
ttcgcgtaca ccattgtcaa catcaatggg caacttcccc aaaggtatcg tgctaggata 1560
cgctatgcct ctactaccaa tctacgaatc tatgttacgg ttgcaggtga acggatcttt 1620
gctggtcagt tcaacaagac aatggatacc ggtgatccac ttacattcca atctttctcc 1680
tacgccacta tcaacaccgc gttcaccttt ccaatgagcc agagcagttt cacagtaggt 1740
gctgatacct tcagttcagg gaacgaagtg tacattgata ggtttgagtt gattccagtt 1800
actgctacac tcgag                                                  1815
```

<210> 8

<211> 1815

<212> DNA

<213> Artificial Sequence

<220>

<223> Synthetic B.t. toxin gene

<400>8

```
atggagaaca acatacagaa tcagtgcgtc ccctacaact gcctcaacaa tcctgaggta 60
gagattctca acgaagagag gtcgacgggc agactgccgc tggacatctc cctgtccctc 120
acacgctttc tcctgtctga gttcgttcca ggtgtgggag tcgcgtttgg cctgttcgac 180
ctcatctggg gcttcatcac tccgtcggat tggagcctct ttcttctcca gatcgagcag 240
ttgattgaac agaggattga gaccttggag aggaaccggg ccatcactac ccttcgtggc 300
ttagcagaca gctacgagat ctacattgaa gccctacggg agtgggaggc caatcccaac 360
aatgcccaac tgcgggaaga tgtgcgtatc cgcttcgcga acaccgatga cgctctgatc 420
accgccatca acaacttcac ccttaccagc ttcgagatac ctctcctctc ggtctatgtt 480
caagctgcga acctgcactt gtcactactg cgcgacgctg tgtcgtttgg gcaagggtgg 540
ggcctggaca tcgctacggt caacaaccac tacaaccgcc tcatcaatct gattcatcga 600
tacacgaaac actgtctgga tacctacaat cagggcttgg agaacctgag aggtacgaac 660
actcgccagt gggccaggtt caaccagttc aggcgcgacc ttacacttac tgtgctggac 720
atagtcgctc tctttccgaa ctacgacgtt cgtacctatc cgatccaaac gagttcccag 780
cttaccaggg agatctacac cagctccgtc attgaagact ctccagtgtc ggcgaacata 840
cccaatggct tcaacagggc tgagttcgga gtccgcccac cccatctcat ggacttcatg 900
aactctctgt tcgtgactgc agagactgtt agatcccaaa cggtgtgggg aggccactta 960
gtcagctcac gcaacacggc gggcaatcgg atcaactttc ctagctacgg ggtgttcaat 1020
cccgggggcg ccatctggat tgcagatgaa gatccgcggc ccttctatcg gaccttgtcc 1080
gatcctgtct tcgtccgagg aggctttggc aaccctcact acgtactcgg tctcaggggc 1140
gtggccttcc aacagactgg tacgaatcac acccgcacat tcaggaactc cgggaccatc 1200
gactctctag acgagatccc gcctcaagac aacagcggcg caccttggaa tgactactcc 1260
cacgtgctga atcatgttac ctttgtgcgc tggccaggtg agatctcagg ctccgactca 1320
tggcgcgcac caatgttctc gtggacgcat cgtagcgcta cccccacaaa caccattgat 1380
ccggagagaa tcactcagat tcccttggtg aaggcccaca cacttcagtc aggcacgaca 1440
gtggtcagag ggccggggtt cacgggagga gacatccttc gacgcactag tggcggacca 1500
ttcgcgtaca ccattgtcaa catcaacggg cagcttcccc aaaggtatcg tgccaggata 1560
cgctatgcct ctactaccaa tctacgcatc tacgttacgg tggcaggcga gcggatcttc 1620
gcgggtcagt tcaacaagac catggacacc ggtgatccac tcacattcca gtctttctcc 1680
tacgccacga tcaacaccgc gttcaccttt ccgatgagcc agagcagctt cacagtaggt 1740
gctgatacct tcagttccgg caacgaagtg tacattgaca ggtttgagtt gatcccagtt 1800
actgccacac tcgag                                                  1815
```

## Description

Background of the Invention

Insects and other pests cost farmers billions of dollars annually in crop losses and in the expense of keeping these pests under control. The losses caused by insect pests in agricultural production environments include decrease in crop yield and reduced crop quality. Increased harvesting costs are also a result of insect infestations.

Insect problems have been partially addressed by cultivation methods, such as crop rotation, and by fertilizing with high levels of phosphate to stimulate the growth of strong root systems by the plants. However, crop rotation can be disrupted by, for example, an emerging two-year diapause (or overwintering) trait of northern corn rootworms. Chemical insecticides are relied upon most heavily to guarantee the desired level of control.

Chemical pesticides have provided an effective method of pest control. However, the public has become concerned about the amount of residual chemicals which might be found in food, ground water, and elsewhere in the environment. Therefore, synthetic chemical pesticides are being increasingly scrutinized for their potential adverse environmental consequences. Some synthetic chemical pesticides can poison the soil and underlying aquifers, pollute surface waters as a result of runoff, and destroy non-target life forms. Some synthetic chemical control agents have the further disadvantage of presenting public safety hazards when they are applied in areas where pets, farm animals, or children may come into contact with them. They can also pose health hazards to the people applying them, especially if the proper application techniques are not followed. Regulatory agencies around the world are restricting and/or banning the uses of many synthetic pesticides, particularly those that are persistent in the environment and that enter the food chain. Stringent new restrictions on the use of pesticides and the elimination of some effective pesticides from the market place could limit economical and effective options for controlling costly pests.

Because of the problems associated with the use of many synthetic chemical pesticides, there exists a clear need to limit the use of these agents and to identify alternative control agents. The replacement of synthetic chemical pesticides, or the combination of these agents with biological pesticides, could reduce the levels of toxic chemicals in the environment.

A biological pesticidal agent that is being used with increasing popularity is the soil microbe *Bacillus thuringiensis* (*B.t.*). The soil microbe *Bacillus thuringiensis* (*B.t.*) is a Gram-positive, spore-forming bacterium. Most strains of *B.t.* do not exhibit pesticidal activity. Some *B.t.* strains produce pesticidal parasporal protein inclusions. These protein inclusions often appear microscopically as distinctively shaped crystals. The shape and type of the crystal inclusions can be used to characterize *B.t.* strains. The "δ-endotoxins" present in the crystalline inclusions, which typically have specific pesticidal activity, are different from exotoxins, which have a non-specific host range.

Commercial use of *B.t.* pesticides was originally restricted to a narrow range of lepidopteran (caterpillar) pests. For example, preparations of the spores and crystals of *B. thuringiensis* subsp. *kurstaki* have been used for many years as commercial insecticides for lepidopteran pests. More recently, however, investigators have discovered *B.t.* pesticides with specificities for a much broader range of pests. For example, *B.t. israelensis* and *morrisoni* have been used commercially to control insects of the orders Diptera and Coleoptera, respectively (Gaertner, F.H. [1989] "Cellular Delivery Systems for Insecticidal Proteins: Living and Non-Living Microorganisms," in *Controlled Delivery of Crop Protection Agents,* R.M. Wilkins, ed., Taylor and Francis, New York and London, 1990, pp. 245-255).

New subspecies of *B.t.* have now been identified, and genes responsible for active δ-endotoxin proteins have been isolated and sequenced (Höfte, H., H.R. Whiteley [1989] *Microbiological Reviews* 52(2):242-255). Höfte and Whiteley classified *B.t.* crystal protein genes into four major classes. The classes were *cry*I (Lepidoptera-specific), *cry*II (Lepidoptera- and Diptera-specific), *cry*III (Coleoptera-specific), and *cry*IV (Diptera-specific). The discovery of strains specifically toxic to other pests has been reported (Feitelson, J.S., J. Payne, L. Kim [1992] *Bio/Technology* 10:271-275). For example, the designations CryV and CryVI have been proposed for two new groups of nematode-active toxins.

Many *Bacillus thuringiensis* δ-endotoxin proteins are composed of two functional segments. These "full-length" proteins are comprised of a protease-resistant core toxin that corresponds to about the first half (the N-terminal portion) of the protein molecule. The three-dimensional structure of a core segment of a CryIIIA *B.t.* δ-endotoxin is known, and it was proposed that all related toxins have that same overall structure (Li, J., J. Carroll, D.J. Ellar [1991] *Nature* 353:815-821). The second half (the C-terminal portion) of the molecule is often referred to as the "protoxin segment." The protoxin segment is believed to participate in toxin crystal formation (Arvidson, H., P.E. Dunn, S. Strand, A.I. Aronson [1989] *Molecular Microbiology* 3:1533-1534; Choma, C.T., W.K. Surewicz, P.R. Carey, M. Pozsgay, T. Raynor, H. Kaplan [1990] *Eur. J. Biochem.* 189:523-527). The full 130 kDa toxin molecule is typically processed to the resistant core segment by proteases in the insect gut. The protoxin segment may thus convey a partial insect specificity for the toxin by limiting the accessibility of the core to the insect by reducing the protease processing of the toxin molecule (Haider, M.Z., B.H. Knowles, D.J. Ellar [1986] *Eur. J. Biochem.* 156:531-540) or by reducing toxin solubility (Aronson, A.I., E.S. Han, W. McGaughey, D. Johnson [1991] *Appl. Environ. Microbial*. 57:981-986).

The 1989 nomenclature and classification scheme of Höfte and Whiteley was based on both the deduced amino acid sequence and the host range of the toxin. That system was adapted to cover 14 different types of toxin genes which were divided into five major classes. A revised nomenclature scheme has been proposed which is based solely on amino acid identity (Crickmore *et al.* [1996] Society for Invertebrate Pathology, 29th Annual Meeting, IIIrd International Colloquium on *Bacillus thuringiensis,* University of Cordoba, Cordoba, Spain, September 1-6, 1996, abstract). The mnemonic "cry" has been retained for all of the toxin genes except cytA and cytB, which remain a separate class. Roman numerals have been exchanged for Arabic numerals in the primary rank, and the parentheses in the tertiary rank have been removed. Current boundaries represent approximately 95% (tertiary rank), 75% (secondary rank), and 48% (primary rank) sequence identity. Many of the original names have been retained, although a number have been reclassified. *See also* "Revisions of the Nomenclature for the *Bacillus thuringiensis* Pesticidal Crystal Proteins," N. Crickmore, D.R. Zeigler, J. Feitelson, E. Schnepf, J. Van Rie, D. Lereclus, J. Baum, and D.H. Dean, *Microbiology and Molecular Biology Reviews* (1998) Vol. 62:807-813; and Crickmore, Zeigler, Feitelson, Schnepf, Van Rie, Lereclus, Baum, and Dean, "*Bacillus thuringiensis* toxin nomenclature" (1999) http://www.biols.susx.ac.uk/Home/Neil_Crickmore/Bt/index.html. That system uses the freely available software applications CLUSTAL W and PHYLIP. The NEIGHBOR application within the PHYLIP package uses an arithmetic averages (UPGMA) algorithm.

Isolating novel *B.t.* isolates and their toxin genes, as well as determining the precise pesticidal range of the toxins, has been a slow empirical process. As a result of extensive research and resource investment, patents have issued for new *B.t.* isolates, toxins, and genes, and for new uses of known *B.t.* toxins and isolates. See Feitelson *et al*., *supra,* for a review. However, the discovery of new *B.t.* isolates and new uses of known *B.t.* isolates and toxins remains an empirical, unpredictable art.

Smulevitch *et al*. ([1991] *FEBS Lett*. 293:25-26), Gleave *et al.* ([1991] *JGM* 138:55-62), and Shevelev *et al*. ([1993] *FEBS Lett*. 336:79-82) describe the characterization of *Cry*9 toxins active against lepidopterans. Lambert *et al.* (Lambert, B., L. Buysse, C. Decock, S. Jansens, C. Piens, B. Saey, J. Seurinck, K. van Audenhove, J. Van Rie, A. Van Vliet, M. Peferoen [1996] *Appl. Environ. Microbiol* 62(1):80-86), and Published PCT applications WO 94/05771 and WO 94/24264 also describe *B.t.* isolates and *Cry*9C toxins active against lepidopteran pests.

U.S. Patent Nos. 5.126,133; 5,188,960; 5,246,852; and 5,691,308 disclose a *Cry*1Fa toxin and gene (81IA) from *B.t.* isolate PS81I. U.S. Patent Nos. 5,527,883; 5,508,264; 5,827,514; and 5,840,554 relate to *Cry*IF chimeric toxins. WO 99/24581 discloses various plant-optimized *cry*1F genes. U.S. Patent No. 5,686,069 discloses a *Cry*1Fb toxin and gene from *B.t*. isolate PS91C2.

With the use of genetic engineering techniques, various approaches for delivering *B.t.* toxins to agricultural environments are under development. The cloning and expression of a *B.t*. crystal protein gene in *Escherichia coli* was described in the published literature more than 15 years ago (Schnepf, H.E., H.R. Whiteley [1981] *Proc. Natl. Acad. Sci. USA* 78:2893-2897.). U.S. Patent No. 4,448,885 and U.S. Patent No. 4,467,036 both disclose the expression of *B.t.* crystal protein in *E. coli.* Recombinant DNA-based *B.t.* products have been produced and approved for use, including the use of plants genetically engineered with *B.t*. genes for insect resistance and the use of stabilized, microbial cells as delivery vehicles *of B.t.* proteins. Various improvements have been achieved by modifying *B.t.* toxins and/or their genes. For example, U.S. Patent Nos. 5,380,831 and 5,567,862 relate to the production of synthetic insecticidal crystal protein genes having improved expression in plants. Thus, isolated *B.t.* endotoxin genes are becoming commercially valuable.

Obstacles to the successful agricultural use of *B.t.* toxins include the development of resistance to *B.t.* toxins by insects. In addition, certain insects can be refractory to the effects of *B.t*. The latter includes insects such as boll weevils and cutworms, which heretofore have demonstrated no apparent sensitivity to most *B.t.* δ-endotoxins.

Cutworms progress through several instars as larvae. Although seedling cutting by later instar larvae produces the most obvious damage and economic loss, leaf feeding commonly results in yield loss in crops such as maize. Upon reaching later instars (third to fourth, for example), larvae begin to cut plants and plant parts, especially seedlings. Because of the shift in feeding behavior, economically damaging populations may build up unexpectedly with few early warning signs. Large cutworms can destroy several seedlings per day, and a heavy infestation can remove entire stands of crops. Cutworms' nocturnal habit and behavior of burrowing into the ground also makes detection and treatment problematic.

The black cutworm (*Agrotis ipsilon* (Hufnagel); Lepidoptera: Noctuidae) is a serious pest of many crops including maize, cotton, cole crops (*Brassica*, broccoli, cabbages, Chinese cabbages), and turf. Secondary host plants include beetroots, *Capsicum* (peppers), chickpeas, faba beans, lettuces, lucerne, onions, potatoes, radishes, rape (canola), rice, soybeans, strawberries, sugarbeet, tobacco, tomatoes, and forest trees. In North America, pests of the genus *Agrotis* feed on clover, corn, tobacco, hemp, onion, strawberries, blackberries, raspberries, alfalfa, barley, beans, cabbage, oats, peas, potatoes, sweetpotatoes, tomato, garden flowers, grasses, lucerne, maize, asparagus, grapes, almost any kind of leaf, weeds, and many other crops and garden plants. Other cutworms in the Tribe Agrotini are pests, in particular those in the genus *Feltia* (*e.g., F. jaculifera* (Guenée); equivalent to *ducens subgothica*) and *Euxoa* (*e.g., E. messoria* (Harris), *E. scandens* (Riley), *E. auxiliaris* Smith, *E. detersa* (Walker), *E. tessellata* (Harris), *E. ochrogaster* (Guenée). Cutworms such as *Peridroma saucia* can also be significant pests. Citrus plants, for example, can also be the target of cutworm attacks (the "citrus cutworm" *Xylomyges curialis* is an example).

Cutworms are also pests outside North America, and the more economically significant pests attack chickpeas, wheat, vegetables, sugarbeet, lucerne, maize, potatoes, turnips, rape, lettuces, strawberries, loganberries, flax, cotton, soybeans, tobacco, beetroots, Chinese cabbages, tomatoes, aubergines, sugarcane, pastures, cabbages, groundnuts, *Cucurbita,* turnips, sunflowers, *Brassica,* onions, leeks, celery, sesame, asparagus, rhubarb, chicory, greenhouse crops, and spinach. The black cutworm *A. ipsilon* occurs as a pest outside North America, including Central America, Europe, Asia, Australia, Africa, India, Taiwan, Mexico, Egypt, and New Zealand.

The main cutworm species in Argentina are *Agrotis malefida, Porosagrotis gypaetiana*, and *Agrotis ipsilon* (also *spelled ypsilon*). These cutworms attack corn, soybean, and sunflower cultivars, for example. These insects can seriously reduce the population of seedlings, and in cases of severe attacks, can totally destroy entire plots.

Cultural controls for *A. ipsilon* such as peripheral weed control can help prevent heavy infestations; however, such methods are not always feasible or effective. Infestations are very sporadic, and applying an insecticide prior to planting or at planting has not been effective in the past. Some baits are available for control of cutworms in crops. To protect turfgrass such as creeping bentgrass, chemical insecticides have been employed. Use of chemical pesticides is a particular concern in turf-covered areas (*e.g.*, golf greens, athletic fields, parks and other recreational areas, professional landscaping, and home lawns) because of the close contact the public has with these areas. Natural products (*e.g.*, nematodes and azadirachtin) generally perform poorly.

To date, *Bacillus thuringiensis* products for control black cutworm have not been widely used to due to their heretofore lack of sufficient effectiveness. The rapid feeding and burrowing behaviors of the cutworm have made them particularly difficult to control with current biologically based pest solutions. For example, *Cry*1A(b) toxins are basically ineffective against cutworms.

## Brief Summary of the Invention

The subject invention relates to the surprising discovery that *Cry*1F proteins are active against cutworms such as the black cutworm (*Agrotis ipsilon*). Thus, the subject invention provides methods for controlling these pests wherein said method comprises contacting said pest with a pesticidal amount of a *Bacillus thuringiensis* toxin comprising at least a pesticidal portion of a *Cry*1F toxin. Therefore, the use of full-length, truncated, and chimeric *Cry*1F proteins and genes is included in the subject invention. In preferred embodiments, the *Cry*1F toxin is a *Cry*1Fa toxin. Wild-type and synthetic *Cry*1F proteins can be used according to the subject invention. Thus, the use of polynucleotides (and/or their complements, preferably their full complements) that hybridize with known *cry*1F genes, preferably the core-toxin encoding portions, are included in this invention. Plant-optimized polynucleotides are used in preferred embodiments.

The subject invention includes the use of transgenic hosts including plant hosts such as corn, cotton, and sunflower. In a preferred embodiment, the subject invention concerns plants cells transformed with at least one polynucleotide sequence of the subject invention such that the transformed plant cells express pesticidal proteins in tissues consumed by the target pests. Such transformation of plants can be accomplished using techniques well known to those skilled in the art and would typically involve modification of the gene to optimize expression of the toxin in plants.

## Brief Description of the Drawings

**Figure 1** shows mass of intact wheat seedlings and relative damage ratings 24 hours after infestation with 4$^{th}$ instar black cutworm larvae and after various treatments.

**Figure 2** shows the fresh weights of wheat seedlings infested with 3$^{rd}$ instar black cutworm larvae 48 hours after infestation and after various treatments.

**Figures 3 and 4** illustrate the results discussed in Example 4.

## Brief Description ofthe Sequences

**SEQ ID NO. 1** is a polynucleotide sequence for a full-length, plant-optimized *cry*IF/*cry*IA(b) hybrid gene designated 1F1AB-PO.

**SEQ ID NO. 2** is an amino acid sequence for a full-length, plant-optimized CryIF/CryIA(b) chimeric toxin. The 1F1AB-PO gene encodes this toxin.

**SEQ ID NO. 3** is a polynucleotide sequence for a truncated, plant-optimized *cry*IF gene designated 1F-T-PO.

**SEQ ID NO. 4** is an amino acid sequence for a truncated, plant-optimized CryIF toxin. The genes designated 1F-T-PO, 1F-7G-PO, and 1F-7Z-PO encode this toxin.

**SEQ ID NO. 5** is the native polynucleotide sequence of the wild-type, full length *B.t.* toxin gene designated 81IA (*cry*IFa).

**SEQ ID NO. 6** is the amino acid sequence of the full length, wild-type *B.t.* toxin designated 81IA (CryIFa).

**SEQ ID NO. 7** is a polynucleotide sequence for a gene designated 1F-7G-PO, which is optimized for expression in cotton.

**SEQ ID NO. 8** is a polynucleotide sequence for a gene designated 1F-7Z-PO, which is optimized for expression in maize.

<u>Detailed Disclosure of the Invention</u>

The subject invention relates to the surprising discovery that *Cry*1F proteins are active against cutworms such as the black cutworm (*Agrotis ipsilon*). Particularly surprising are the findings that 3rd instar, and greater, larvae are controlled by the toxins and genes according to the subject invention. Controlling first and second instar cutworm larvae is much less important than controlling later instars. While it was known that *Cry*I toxins are active against lepidopterans generally, cutworms were thought to be recalcitrant to *B.t.* toxins generally, as discussed in more detail above in the Background section.

The subject invention includes the use of recombinant hosts and provides plant-optimized polynucleotide sequences These polynucleotide sequences include plant-optimized genes designated 1F1AB-PO, 1F-T-PO, 1F-7G-PO, and 1F-7Z-PO. These genes are disclosed in WO 99/24581. Preferred plant hosts include corn, soybeans, cotton, wheat, canola, and sunflower.

In some embodiments of the subject invention, genes encode a *Cry*IF toxin that is truncated compared to the full length *Cry*IF toxin. The truncated toxins of the subject invention are typically missing all or a portion of the protoxin segment. Also, the truncated genes of the subject invention can be used for the production of chimeric genes and proteins. One example is the plant-optimized gene comprising a *cry*IF portion and a *cry*IA(b) portion, wherein the hybrid gene encodes a chimeric toxin. Preferred chimeric genes and toxins are disclosed in U.S. Patent Nos. 5,527,883 and 5,840,554. Other chimeric genes and toxins, which can be used according to the subject invention, are disclosed in U.S. Patent Nos. 5,508,264 and 5,827,514. In a preferred embodiment, the *Cry*IF portion of the chimeric toxin is itself pesticidal. Fusion toxins can also be used according to the subject invention.

In a preferred embodiment, the subject invention concerns plants cells transformed with at least one polynucleotide sequence such that the transformed plant cells express pesticidal toxins in tissues consumed by the target pests which thereby contact the pesticidal protein. Such transformation of plants can be accomplished using techniques well known to those skilled in the art and would typically involve modification of the gene to optimize expression of the toxin in plants.

It should be apparent to a person skilled in this art that, given the sequences of the genes as set forth herein, the genes of the subject invention can be obtained through several means. In preferred embodiments, the subject genes may be constructed synthetically by using a gene synthesizer, for example. The specific genes exemplified herein can also be obtained by modifying, according to the teachings of the subject invention, certain wild-type genes (for example, by point-mutation techniques) from certain isolates deposited at a culture depository as discussed below. For example, a wild-type *cry*IF gene can be obtained from *B.t.* isolate PS81I. Likewise, the *cry*IA(b) portions of the hybrid genes of the subject invention can be produced synthetically or can be derived by modifying wild-type genes. CryIA(b) toxins and genes have been described in, for example, Höfte *et al.* (1986) *Eur. J. Biochem*. 161:273; Geiser *et al.* (1986) *Gene* 48:109; and Haider *et al.* (1988) *Nucleic Acids Res.* 16:10927. Clones and additional wild-type isolates are discussed in more detail, above, in the section entitled "Background of the Invention" and in the list, below.

Cultures discussed in this application have been deposited, in accordance with the Budapest Treaty, in the Agricultural Research Service Patent Culture Collection (NRRL), Northern Regional Research Center, 1815 North University Street, Peoria, Illinois 61604, USA. The deposited strains listed below are disclosed in the patent references as discussed above in the section entitled "Background of the Invention."

| Subculture | Accession Number | Deposit Date |
|---|---|---|
| *B.t.* PS81I | NRRL B-18484 | April 19, 1989 |
| *E. coli* (NM522) (pMYC1603) (81IA) | NRRL B-18517 | June 30, 1989 |

It should be understood that the availability of a deposit does not constitute a license to practice the subject invention in derogation of patent rights granted by governmental action.

<u>Genes and toxins</u>. The polynucleotides of the subject invention can be used to form complete "genes" to encode proteins or peptides in a desired host cell. For example, as the skilled artisan would readily recognize, some of the polynucleotides in the attached sequence listing are shown without stop codons. Also, the subject polynucleotides can be appropriately placed under the control of a promoter in a host of interest, as is readily known in the art.

As the skilled artisan would readily recognize, DNA typically exists in a double-stranded form. In this arrangement, one strand is complementary to the other strand and vice versa. As DNA is replicated in a plant (for example) additional, complementary strands of DNA are produced. Thus, the subject invention includes the use of the exemplified polynucleotides shown in the attached sequence listing and the complementary strands thereof. The "coding strand" is often used in the art to refer to the strand that binds with the anti-sense strand. In order to express a protein *in vivo*, a strand of DNA is typically transcribed into a complementary strand of mRNA which is used as the template for translating the protein. The mRNA is actually transcribed from the "anti-sense" strand of DNA. The "sense" or "coding" strand has a series of codons (a codon is three nucleotides that can be read three-at-a-time to yield a particular amino acid) that can be read as an open reading frame (ORF) to form a protein or peptide of interest. RNA and PNA (peptide nucleic acids) that are functionally equivalent to the exemplified DNA are included in the subject invention.

Genes and toxins of the subject invention can be identified, obtained, and characterized by using oligonucleotide probes, for example. Probes are detectable nucleotide sequences. The specifically exemplified polynucleotides of the subject invention, including portions thereof that are sufficient to encode an active toxin, can, themselves, be used as probes. Probes may be DNA, RNA, or PNA (peptide nucleic acid). These sequences may be detectable by virtue of an appropriate label including or may be made inherently fluorescent, for example, as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G.H., M.M. Manak (1987) *DNA Probes,* Stockton Press, New York, NY., pp. 169-170. For example, as stated therein, hybridization conditions of high stringency can be achieved by first washing with 2x SSC (Standard Saline Citrate)/0.1% SDS (Sodium Dodecyl Sulfate) for 15 minutes at room temperature. Two washes are typically performed. Higher stringency can be achieved by lowering the salt concentration and/or by raising the temperature. For example, the above hybridization step can be followed by washing with 0.1x SSC/0.1% SDS for 15 minutes at room temperature, which in turn can be followed by washing with 0.1x SSC/0.1% SDS for 30 minutes at 55°C. The temperatures used for these steps can also be used with other protocols discussed herein (where SSPE is used in place of SSC, for example) as would be known in the art. The 2x SSC/0.1% SDS can be prepared by adding 50 ml of 20x SSC and 5 ml of 10% SDS to 445 ml of water. 20x SSC can be prepared by combining NaCl (175.3 g / 0.150 M), sodium citrate (88.2 g / 0.015 M), and water to 1 liter, followed by adjusting pH to 7.0 with 10 N NaOH. 10% SDS can be prepared by dissolving 10 g of SDS in 50 ml of autoclaved water, diluting to 100 ml, and aliquotting.

Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures.

As used herein "stringent" conditions for hybridization refers to conditions which achieve the same, or about the same, degree of specificity of hybridization as the conditions employed by the current applicants. Specifically, hybridization of immobilized DNA on Southern blots with 32P-labeled gene-specific probes was performed by standard methods (Maniatis, T., E.F. Fritsch, J. Sambrook [1982] *Molecular Cloning: A Laboratory Manual,* Cold Spring Harbor Laboratory, Cold Spring Harbor, NY.). In general, hybridization and subsequent washes were carried out under stringent conditions that allowed for detection of target sequences. For double-stranded DNA gene probes, hybridization was carried out overnight at 20-25° C below the melting temperature (Tm) of the DNA hybrid in 6X SSPE, 5X Denhardt's solution, 0.1% SDS, 0.1 mg/ml denatured DNA. The melting temperature is described by the following formula (Beltz, G.A., K.A. Jacobs, T.H. Eickbush, P.T. Cherbas, and F.C. Kafatos [1983] *Methods of Enzymology*, R. Wu, L. Grossman and K. Moldave [eds.] Academic Press, New York 100:266-285).

Tm=81.5° C+16.6 Log[Na+]+0.41(%G+C)-0.61(%formamide)-600/length of duplex in base pairs.

Washes are typically carried out as follows:

(1) Twice at room temperature for 15 minutes in 1X SSPE, 0.1% SDS (low stringency wash).
(2) Once at Tm-20°C for 15 minutes in 0.2X SSPE, 0.1% SDS (moderate stringency wash).

For oligonucleotide probes, hybridization was carried out overnight at 10-20°C below the melting temperature (Tm) of the hybrid in 6X SSPE, 5X Denhardt's solution, 0.1% SDS, 0.1 mg/ml denatured DNA. Tm for oligonucleotide probes was determined by the following formula:

Tm (° C)=2(number T/A base pairs) +4(number G/C base pairs)

(Suggs, S.V., T. Miyake, E.H. Kawashime, M.J. Johnson, K. Itakura, and R.B. Wallace [1981] *ICN-UCLA Symp. Dev.*

*Biol. Using Purified Genes,* D.D. Brown [ed.], Academic Press, New York, 23:683-693).

Washes were typically carried out as follows:

(1) Twice at room temperature for 15 minutes 1X SSPE, 0.1% SDS (low stringency wash).

(2) Once at the hybridization temperature for 15 minutes in 1X SSPE, 0.1% SDS (moderate stringency wash).

Modification of genes and toxins. The genes and toxins useful according to the subject invention include not only the specifically exemplified sequences, but also include portions and/or fragments (including internal and/or terminal deletions compared to the full-length proteins), variants, mutants, substitutes (proteins having substituted amino acids), chimerics, and fusion proteins which retain the characteristic pesticidal activity of the proteins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

Genes can be modified, and variations of genes may be readily constructed, using standard techniques. For example, techniques for making point mutations are well known in the art. Also, U.S. Patent No. 5,605,793, for example, describes methods for generating additional molecular diversity by using DNA reassembly after random fragmentation. Fragments of full-length genes can be made using commercially available endonucleases, and exonucleases can be used according to standard procedures. For example, enzymes such as *Bal*31 or site-directed mutagenesis can be used to systematically cut off nucleotides from the ends of these genes. Also, genes which encode active fragments may be obtained using a variety of restriction enzymes. Proteases may be used to directly obtain active fragments of these toxins.

Equivalent toxins and/or genes encoding these equivalent toxins can be derived from *B.t.* isolates and/or DNA libraries using the teachings provided herein. There are a number of methods for obtaining the pesticidal toxins of the instant invention. For example, antibodies to the pesticidal toxins disclosed and claimed herein can be used to identify and isolate other toxins from a mixture of proteins. Specifically, antibodies may be raised to the portions of the toxins which are most constant and most distinct from other *B.t.* toxins. These antibodies can then be used to specifically identify equivalent toxins with the characteristic activity by immunoprecipitation, enzyme linked immunosorbent assay (ELISA), or western blotting. Antibodies to the toxins disclosed herein, or to equivalent toxins, or fragments of these toxins, can readily be prepared using standard procedures in this art. The genes which encode these toxins can then be obtained from the microorganism.

Because of the redundancy of the genetic code, a variety of different DNA sequences can encode the same amino acid sequences. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments retaining pesticidal activity are also included in this definition.

Equivalent toxins will have amino acid similarity (and/or homology) with an exemplified toxin. The amino acid similarity/identity will typically be greater than 60%, preferably greater than 75%, more preferably greater than 80%, even more preferably greater than 90%, and can be greater than 95%. Preferred polynucleotides and proteins of the subject invention can also be defined in terms of more particular identity and/or similarity ranges. For example, the identity and/or similarity can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% as compared to a sequence exemplified herein. Unless otherwise specified, as used herein percent sequence identity and/or similarity of two nucleic acids is determined using the algorithm of Karlin and Altschul (1990), *Proc. Natl. Acad. Sci. USA* 87:2264-2268, modified as in Karlin and Altschul (1993), *Proc. Natl. Acad. Sci. USA* 90:5873-5877. Such an algorithm is incorporated into the NBLAST and XBLAST programs of Altschul *et al.* (1990), *J. Mol. Biol.* 215:402-410. BLAST nucleotide searches are performed witht he NBLAST program, score = 100, wordlength = 12, to obtain nucleotide sequences with the desired percent sequence identity. To obtain gapped alignments for comparison purposes, Gapped BLAST is used as described in Altschul *et al.* (1997), *Nucl. Acids Res.* 25:3389-3402. When utilizing BLAST and Gapped BLAST programs, the default parameters of the respective programs (NBLAST and XBLAST) are used. *See* http://www.ncbi.nih.gov. The identity scores can also be calculated using the methods and algorithms of Crickmore *et al.* as described in the Background section, above.

The amino acid homology will be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 1 provides a listing of examples of amino acids belonging to each class.

Table 1.

| Class of Amino Acid | Examples of Amino Acids |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the ability of plants to express the subject DNA sequences or from the biological activity of the toxin.

As used herein, reference to "isolated" polynucleotides and / or "purified" toxins refers to these molecules when they are not associated with the other molecules with which they would be found in nature; this would include their use in plants. Thus, reference to "isolated" and/or "purified" signifies the involvement of the "hand of man" as described herein.

While the subject invention provides specific embodiments of synthetic genes, other genes that are functionally equivalent to the genes exemplified herein can also be used to transform hosts, preferably plant hosts. Additional guidance for the production of synthetic genes can be found in, for example, U.S. Patent No. 5,380,831.

Recombinant hosts. The toxin-encoding genes of the subject invention can be introduced into a wide variety of microbial or plant hosts. In preferred embodiments, expression of the toxin gene results, directly or indirectly, in the intracellular production and maintenance of the pesticide. When transgenic/recombinant/transformed host cells are ingested by the pest, the pests will ingest the toxin. This is the preferred manner in which to cause contact of the pest with the toxin. The result is a control (killing or making sick) of the pest.

In some embodiments of the subject invention, transformed microbial hosts can be used in preliminary steps for preparing precursors, for example, that will eventually be used to transform, in preferred embodiments, plant cells and plants so that they express the toxins encoded by the genes of the subject invention. Microbes transformed and used in this manner are within the scope of the subject invention. Recombinant microbes may be, for example, *B.t.*, *E. coli,* or *Pseudomonas.*

The production of various recombinant organisms can be made by those skilled in the art using standard techniques. Materials necessary for these transformations are disclosed herein or are otherwise readily available to the skilled artisan. A wide variety of methods are available for introducing a *B.t.* gene encoding a toxin into the target host under conditions which allow for stable maintenance and expression of the gene. These methods are well known to those skilled in the art and are described, for example, in United States Patent No. 5,135,867, which is incorporated herein by reference.

The toxin-encoding genes of the subject invention can be introduced, via a suitable vector, into a wide variety of microbial and plant hosts. There are many crops of interest, such as corn, wheat, rice, cotton, soybeans, and sunflowers. Certain genes of the subject invention are particularly well suited for providing stable maintenance and expression, in the transformed plant, of the gene expressing the polypeptide pesticide, and, desirably, provide for improved protection of the pesticide from environmental degradation and inactivation. Expression of the toxin gene results, directly or indirectly, in the intracellular production and maintenance of the pesticide proteins. Thus, the target pest can contact the pesticidal proteins by ingesting plant tissue containing the pesticidal proteins, which are toxic to the pest. The result is control of the pest. Alternatively, suitable microbial hosts, *e.g., Pseudomonas fluorescens*, can be applied to the site of the pest, where some of which can proliferate, and are ingested by the target pests. The microbe hosting the toxin gene can be treated under conditions that prolong the activity of the toxin and stabilize the cell. The treated cell, which retains the toxic activity, then can be applied to the environment of the target pest.

Where the *B.t.* toxin gene is introduced via a suitable vector into a microbial host, and said host is applied to the environment in a living state, certain host microbes should be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, *e.g*., genera *Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc*, and *Alcaligenes;* fungi, particularly yeast, *e.g*., genera *Saccharomyces, Cryptococcus, Kluyveromyces, Sporobolomyces, Rhodotorula,* and *Aureobasidium*. Of particular interest are such phytosphere bacterial species as *Pseudomonas syringae, Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus,* and *Azotobacter vinlandii*; and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans*. Of particular interest are the pigmented microorganisms.

A wide variety of ways are available for introducing a *B.t*. gene encoding a toxin into the target host under conditions which allow for stable maintenance and expression of the gene. These methods are well known to those skilled in the art and are described, for example, in United States Patent No. 5,135,867, which is incorporated herein by reference.

<u>Treatment of cells.</u> As mentioned above, *B.t*. or recombinant cells expressing a *B.t.* toxin can be treated to prolong the toxin activity and stabilize the cell. The pesticide microcapsule that is formed comprises the *B.t.* toxin within a cellular structure that has been stabilized and will protect the toxin when the microcapsule is applied to the environment of the target pest. Suitable host cells may include either prokaryotes or eukaryotes, normally being limited to those cells which do not produce substances toxic to higher organisms, such as mammals. However, organisms which produce substances toxic to higher organisms could be used, where the toxic substances are unstable or the level of application sufficiently low as to avoid any possibility of toxicity to a mammalian host. As hosts, of particular interest will be the prokaryotes and the lower eukaryotes, such as fungi.

The cell will usually be intact and be substantially in the proliferative form when treated, rather than in a spore form, although in some instances spores may be employed.

Treatment of the microbial cell, *e.g*., a microbe containing the *B.t.* toxin gene, can be by chemical or physical means, or by a combination of chemical and/or physical means, so long as the technique does not deleteriously affect the properties of the toxin, nor diminish the cellular capability of protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as glutaraldehyde; anti-infectives, such as zephiran chloride and cetylpyridinium chloride; alcohols, such as isopropyl and ethanol; various histologic fixatives, such as Lugol iodine, Bouin's fixative, various acids and Helly's fixative (See: Humason, Gretchen L., *Animal Tissue Techniques,* W.H. Freeman and Company, 1967); or a combination of physical (heat) and chemical agents that preserve and prolong the activity of the toxin produced in the cell when the cell is administered to the host environment. Examples of physical means are short wavelength radiation such as gamma-radiation and X-radiation, freezing, UV irradiation, lyophilization, and the like. Methods for treatment of microbial cells are disclosed in United States Patent Nos. 4,695,455 and 4,695,462, which are incorporated herein by reference.

The cells generally will have enhanced structural stability which will enhance resistance to environmental conditions. Where the pesticide is in a proform, the method of cell treatment should be selected so as not to inhibit processing of the proform to the mature form of the pesticide by the target pest pathogen. For example, formaldehyde will crosslink proteins and could inhibit processing of the proform of a polypeptide pesticide. The method of treatment should retain at least a substantial portion of the bio-availability or bioactivity of the toxin.

Characteristics of particular interest in selecting a host cell for purposes of production include ease of introducing the *B.t.* gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

<u>Growth of cells.</u> The cellular host containing the *B.t.* insecticidal gene may be grown in any convenient nutrient medium, preferably where the DNA construct provides a selective advantage, providing for a selective medium so that substantially all or all of the cells retain the *B.t*. gene. These cells may then be harvested in accordance with conventional ways. Alternatively, the cells can be treated prior to harvesting.

The *B.t.* cells of the invention can be cultured using standard art media and fermentation techniques. Upon completion of the fermentation cycle the bacteria can be harvested by first separating the *B.t*. spores and crystals from the fermentation broth by means well known in the art. The recovered *B.t*. spores and crystals can be formulated into a wettable powder, liquid concentrate, granules or other formulations by the addition of surfactants, dispersants, inert carriers, and other components to facilitate handling and application for particular target pests. These formulations and application procedures are all well known in the art.

Formulations. Formulated bait granules containing an attractant and spores and crystals of the *B.t.* isolates, or recombinant microbes comprising the genes obtainable from the *B.t.* isolates disclosed herein, can be applied to the soil. Formulated product can also be applied as a seed-coating or root treatment or total plant treatment at later stages of the crop cycle. Plant and soil treatments *of B.t.* cells may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

As would be appreciated by a person skilled in the art, the pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the pest, *e.g.,* soil and foliage, by spraying, dusting, sprinkling, or the like.

Mutants. Mutants of the isolates of the invention can be made by procedures well known in the art. For example, an asporogenous mutant can be obtained through ethylmethane sulfonate (EMS) mutagenesis of an isolate. The mutants can be made using ultraviolet light and nitrosoguanidine by procedures well known in the art.

A smaller percentage of the asporogenous mutants will remain intact and not lyse for extended fermentation periods; these strains are designated lysis minus (-). Lysis minus strains can be identified by screening asporogenous mutants in shake flask media and selecting those mutants that are still intact and contain toxin crystals at the end of the fermentation. Lysis minus strains are suitable for a cell treatment process that will yield a protected, encapsulated toxin protein.

To prepare a phage resistant variant of said asporogenous mutant, an aliquot of the phage lysate is spread onto nutrient agar and allowed to dry. An aliquot of the phage sensitive bacterial strain is then plated directly over the dried lysate and allowed to dry. The plates are incubated at 30°C. The plates are incubated for 2 days and, at that time, numerous colonies could be seen growing on the agar. Some of these colonies are picked and subcultured onto nutrient agar plates. These apparent resistant cultures are tested for resistance by cross streaking with the phage lysate. A line of the phage lysate is streaked on the plate and allowed to dry. The presumptive resistant cultures are then streaked across the phage line. Resistant bacterial cultures show no lysis anywhere in the streak across the phage line after overnight incubation at 30°C. The resistance to phage is then reconfirmed by plating a lawn of the resistant culture onto a nutrient agar plate. The sensitive strain is also plated in the same manner to serve as the positive control. After drying, a drop of the phage lysate is placed in the center of the plate and allowed to dry. Resistant cultures showed no lysis in the area where the phage lysate has been placed after incubation at 30°C for 24 hours.

All patents, patent applications, provisional applications, and publications referred to or cited herein are incorporated by reference in their entirety to the extent they are not inconsistent with the explicit teachings of this specification.

Following are examples which illustrates procedures for practicing the invention. These examples should not be construed as limiting.

## Example 1 - Bioassay of Cry1F against black cutworm (*Agrotis ipsilon;* Lepidoptera: Noctuidae) larvae

Biological activity of Cry1F against black cutworm (BCW) larvae was determined using standard bioassay procedures using MR872 (a *Pseudomonas fluorescens* clone expressing a *cry*1Fa/*cry*1Ab chimeric gene disclosed in U.S. Paatent No. 5,840,554) lyophilized powder preparations. BCW bioassays were conducted by incorporating test samples into artificial diet and challenging larvae with treated and untreated diet. All assays were conducted with BCW artificial diet (BioServ Corporation, Frenchtown, NJ). Diet incorporation tests were conducted by mixing the samples with artificial diet (cooled first to 55°C or below) at a rate of 6 mL suspension plus 54 mL diet. Multiple treatment concentrations were generated by serial dilution. After vortexing, this mixture was poured into plastic trays with compartmentalized 3-ml (Nutrend Container Corporation, Jacksonville, FL) at a rate of approximately 30 ml diet per 24-well tray (each well filled halfway). Nutrend trays with larger wells were used for bioassays with insects in the third instar or older; these also are filled approximately halfway with artificial diet. A water blank containing no test material served as the control. Following at least 30 minutes to allow diet to cool and set, larvae (French Ag Resources, Lamberton, MN) were placed onto the diet mixture, one larva per well. Wells were then sealed with Mylar sheeting (ClearLam Packaging, IL) using a tacking iron, and several pinholes were made in each well to provide gas exchange. Larvae were held at 25°C for 6 days in a 14:10 (light:dark) holding room. Mortality and stunting were recorded after approximately six days. For $LC_{50}$ bioassays, a minimum of 3 replicates were run, each with 5-7 doses and approximately 24 insects/dose. Results are shown in Table 2 (Bioassays against first- and third-instar *Agrotis ipsilon* larvae with MR872 Cry1F lyophilized powder preparation).

Table 2

| Instar | # test dates | $LC_{10}$ | $LC_{50}$ | $LC_{90}$ | slope | # test insects | # control insects | % control mortality |
|---|---|---|---|---|---|---|---|---|
| 1 | 5 | 9 | 46 | 249 | 1.8 | 790 | 220 | 6 |
| 3 | 3 | 4 | 17 | 67 | 2.2 | 249 | 75 | 0 |

Example 2 - Efficacy of Cry1F bait against black cutworm larvae

Cry1F was tested for activity against black cutworm larvae using a bait formulation. MR872 clones were mixed with commercial bait (SoilServ, Salinas, CA) and the resulting mixture was spread across the soil surface of potted wheat seedlings (Feekes growth stage 1.5) at a rate of 50 lb bait/acre. Two concentrations of Cry1F were tested by adding different amounts of toxin to the bait, yielding rates equivalent to 6.25g and 12.5 g of Cry1F toxin/acre. The plants were then infested with 3$^{rd}$ or 4$^{th}$ instar larvae at a rate of approximately 1 insect per 5 plants, and plant damage was measured after 24-48 hours. The damage rating scale runs from 0-4 with 0 being plants cut totally to the ground, and 4 being no visible damage. Controls included plants with no bait added ("no bait"), bait added but without toxin ("blank bait"), and plants with no bait or insects ("control"). Results are shown in Figures 1 and 2.

Example 3 — Efficacy of Transgenic *Cry*1F Sunflower Plants Against *Agrotis ypsilon* as Measured by Insect Growth and Mortality, and Plant Damage

Plant treatments:

- Control seedlings at V2 stage
- *B.t.* seedlings at V2 stage (donors with fixed gene pattern); these plants expressed a gene substantially as shown in SEQ ID NO:3.

Insect: *Agrotis ypsilon*. Second instar larvae (third laboratory generation).
Trial: *B.t.* seedlings: five repetitions, five seedlings per repetition, one larva per seedling. Control: five repetitions, five seedlings per repetition, one larva per seedling.
Protocol:

- Each repetition consisted of five seeds that were planted in transparent rectangular trays (15cm x 25cm) and covered to allow confinement of larval insects.
- Each repetition was infested with five larvae. Trays were kept in a chamber at 25 °C and 75% humidity. Evaluation was done at 24, 48, 72, and 96 hours after infestation.
- Evaluation

    1. Plants — categorized as undamaged, damaged (bitten stem, cotyledons or leaves) or cut below the cotyledons. A rating was also given to each repetition: 1 (undamaged plants or with the stem with some bites) to 9 (all plants cut and more than 80% of eaten tissue).
    2. Insects — 96 hours after infestation dead larvae were counted, and surviving larvae were weighed.
    3. Data were analyzed via contingency table analysis.

Results: Pictures are available that show repetition four (48 and 96 hours after infestation). Data are reported in the following tables, which show evaluation of repetitions 24, 48, 72 and 96 hours after infestation with *Agrotis ypsilon*.

Table 3.

| 24 hours after infestation. | | | | | | | | |
|---|---|---|---|---|---|---|---|---|
| Rep. | No. Undamaged Plants | | No. Damaged Plants | | No. Cut Plants | | Rating | |
| | ***B.t.*** | **control** | ***B.t.*** | **control** | ***B.t.*** | **control** | ***B.t.*** | **control** |
| 1 | 2 | 2 | 2 | 0 | 1 | 3 | 1 | 5 |
| 2 | 3 | 3 | 0 | 1 | 2 | 1 | 3 | 4 |
| 3 | 3 | 2 | 2 | 0 | 0 | 3 | 2 | 5 |
| 4 | 3 | 1 | 0 | 2 | 0 | 2 | 2 | 3 |
| 5 | 4 | 2 | 0 | 2 | 1 | 1 | 2 | 3 |

Table 4.

| 48 hours after infestation. | | | | | | | | |
|---|---|---|---|---|---|---|---|---|
| Rep. | No. Undamaged Plants | | No. Damaged Plants | | No. Cut Plants | | Rating | |
| | ***B.t.*** | **control** | ***B.t.*** | **control** | ***B.t.*** | **control** | ***B.t.*** | **control** |
| 1 | 2 | 1 | 1 | 1 | 2 | 3 | 3 | 7 |
| 2 | 2 | 0 | 1 | 2 | 2 | 3 | 3 | 7 |
| 3 | 1 | 1 | 1 | 0 | 3 | 4 | 3 | 7 |
| 4 | 2 | 1 | 0 | 0 | 3 | 4 | 4 | 7 |
| 5 | 2 | 1 | 1 | 1 | 2 | 3 | 3 | 7 |

Table 5.

| 72 hours after infestation. | | | | | | | | |
|---|---|---|---|---|---|---|---|---|
| Rep. | No. Undamaged Plants | | No. Damaged Plants | | No. Cut Plants | | Rating | |
| | ***B.t.*** | **control** | ***B.t.*** | **control** | ***B.t.*** | **control** | ***B.t.*** | **control** |
| 1 | 2 | 0 | 1 | 0 | 3 | 5 | 3 | 9 |
| 2 | 0 | 0 | 3 | 0 | 2 | 5 | 4 | 9 |
| 3 | 1 | 0 | 1 | 0 | 3 | 5 | 5 | 9 |
| 4 | 1 | 0 | 0 | 0 | 4 | 5 | 6 | 9 |
| 5 | 2 | 0 | 0 | 0 | 5 | 5 | 5 | 9 |

Table 6.

| 96 hours after infestation. | | | | | | | | | |
|---|---|---|---|---|---|---|---|---|---|
| Rep. | No. Undamaged Plants | | No. Damaged Plants | | No. Cut Plants | | Rating | | No. Dead Larvae |
| | ***B.t.*** | **control** | ***B.t.*** | **control** | ***B.t.*** | **control** | ***B.t.*** | **control** | |
| 1 | 3 | 0 | 0 | 0 | 2 | 5 | 3 | 9 | 4 |
| 2 | 0 | 0 | 2 | 0 | 3 | 5 | 7 | 9 | 0 |

Table 6. (continued)

| 96 hours after infestation. | | | | | | | | | |
|---|---|---|---|---|---|---|---|---|---|
| **Rep.** | **No. Undamaged Plants** | | **No. Damaged Plants** | | **No. Cut Plants** | | **Rating** | | **No. Dead Larvae** |
| | ***B.t.*** | **control** | ***B.t.*** | **control** | ***B.t.*** | **control** | ***B.t.*** | **control** | |
| 3 | 1 | 0 | 0 | 0 | 4 | 5 | 5 | 9 | 0 |
| 4 | 0 | 0 | 0 | 0 | 5 | 5 | 6 | 9 | 0 |
| 5 | 1 | 0 | 0 | 0 | 4 | 5 | 6 | 9 | 0 |

Table 7.

| Larval weight (mg) 96 hours after infestation* | | | | | | | |
|---|---|---|---|---|---|---|---|
| **Repetition** | | **Larvae per Repetition** | | | | | |
| | | **1** | **2** | **3** | **4** | **5** | **Mean** |
| 2 | *B.t.* | 100 | 95 | 65 | 115 | 75 | 90 |
| | Control | 230 | 170 | 205 | 175 | 175 | 191 |
| 3 | *B.t.* | 150 | 135 | 85 | 115 | 120 | 121 |
| | Control | 215 | 200 | 90 | 120 | 160 | 157 |
| 4 | *B.t.* | 110 | 80 | 110 | 85 | 150 | 107 |
| | Control | 170 | 160 | 185 | 200 | 155 | 174 |
| 5 | *B.t.* | 120 | 65 | 95 | 60 | 45 | 77 |
| | Control | 120 | 190 | 210 | 255 | 110 | 177 |

*Four repetitions because in one repetition larvae died

Conclusion: The *B.t.* seedlings show activity towards *Agrotis ypsilon* at a high level of infestation (1 larva/seedling). This activity is sufficient to adversely affect the growth of the cutworms so that control is achieved.

Example 4 — Black Cutworm (BCW) Control with New Cry1F Maize Events

Summary. The ability of ten Cry1F maize events (expressing substantially the polynucleotide of SEQ ID NO: 3) to control Black Cutworm (*Agrotis ipsilon* (Hufnagel)) under field conditions was compared to two non-Bt hybrids (Control 1 and Control 2), a hybrid expressing *Cry*1Ab, and a hybrid expressing the *Cry*9C protein for purposes of comparison. All the *Cry*1F events provided good protection against stand loss in comparison to the non-Bt hybrids.

Materials and methods. Twenty-five seeds for each of the entries were planted using an Almaco cone planter on Day 1. Four replications were planted — each randomized separately. Rows were in pairs, with an unplanted walkway between each pair of rows. Each plot was enclosed in a galvanized steel barrier that is 8 inches high, 2.5 feet wide and 6 feet long. The barriers were pounded into the ground about 2 inches deep on Days 13 and 14. The rims of the barriers were treated with petroleum jelly to discourage larval escape. Wheat straw was scattered inside the barriers to provide shelter for the cutworms. Shortly after emergence, entry plants with *Cry*1F were tested with ELISA strips and the stands were thinned to 10 plants by the removal of non-expressing plants or surplus expressing plants. In three of the plots, the final stand was 8 or 9 plants. The non-*Cry*1F's were also thinned to 10 plants. A small plastic stake was placed behind each plant so that it would be apparent that a plant had disappeared, if this occurred. On day 15, when the plants reached late V1 or early V2, three third instar larval black cutworms were placed by each plant. A second infestation of 2 fourth instar BCW per plant was made on Day 19.

Plots were examined each day from Day 16 to Day 26. An additional rating was made on Day 29, and the final rating was made on Day 34. Any plant that received damage was marked with a small plastic stake in front of it and the type of damage was recorded on a data sheet. At the end of the trial the final stand count was taken.

Results. A moderate level of black cutworm pressure was achieved in this trial, resulting in stand reduction in some of the non-Bt entries. For unknown reasons, there was a tendency in this trial for the BCW to dead-heart the plants instead of cutting them. For this reason, the data on damaged plants were also compared.

Table 8 shows plant type and the percent Stand Reduction (%SR) and percent damaged plants (%Dam) for each type of plant. Stand Reduction means that the plant was killed. Damaged plants had obvious feeding damage but did not lose their growing point.

Table 8.

| **Treatment/*Cry*1F event** | **%SR** | **%Dam** |
|---|---|---|
| Non-*B.t.* Control 1 | 47.5 | 60 |
| Non-*B.t.* Control 2 | 32.5 | 45 |
| *Cry*9C | 7.5 | 20 |
| *Cry*1Ab | 15 | 43 |
| 308 | 2.5 | 5 |
| 188 | 0 | 5 |
| 218 | 7.5 | 15 |
| 386 | 7.5 | 15 |
| 538 | 2.5 | 18 |
| 778 | 2.5 | 15 |
| 366 | 3.1 | 11 |
| 663 | 5 | 18 |
| 058 | 2.8 | 9 |
| 227 | 0 | 5 |

These results are shown graphically in Figures 3 and 4.

Although no entries were without damage, this is not surprising because the caterpillars have to eat the plants to die. However, in summary, photographs are available that clearly show slightly affected *Cry*1F plants next to completely cut control plants.

Example 5 - Insertion of Toxin Genes Into Plants

One aspect of the subject invention is the transformation of plants with the subject polynucleotide sequences encoding insecticidal toxins. The transformed plants are resistant to attack by the target pest. Preferred genes for use according to the subject invention are optimized for use in plants. Examples of cutworm-active toxins and genes for use according to the subject invention are shown in SEQ ID NOS. 1-8. The protein and gene of SEQ ID NOS. 1 and 2 are preferred.

Obviously, a promoter region capable of expressing the gene in a plant is needed. Thus, for *in planta* expression, the DNA of the subject invention is under the control of and operably linked to an appropriate promoter region. Techniques for obtaining *in planta* expression by using such constructs is known in the art.

Genes encoding pesticidal toxins, as disclosed herein, can be inserted into plant cells using a variety of techniques which are well known in the art. For example, a large number of cloning vectors comprising a replication system in *E. coli* and a marker that permits selection of the transformed cells are available for preparation for the insertion of foreign genes into higher plants. The vectors comprise, for example, pBR322, pUC series, M13mp series, pACYC184, etc. Accordingly, the sequence encoding the *B.t.* toxin can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids.

Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516; Hoekema (1985) In: *The Binary Plant Vector System,* Offset-durkkerij Kanters B.V., Alblasserdam, Chapter 5; Fraley *et al., Crit. Rev. Plant Sci.* 4:1-46; and An *et al.* (1985) *EMBO J*. 4:277-287.

Once the inserted DNA has been integrated in the genome, it is relatively stable there and, as a rule, does not come out again. It normally contains a selection marker that confers on the transformed plant cells resistance to a biocide or an antibiotic, such as kanamycin, G 418, bleomycin, hygromycin, or chloramphenicol, *inter alia.* The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, biolistics (microparticle bombardment), or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the *vir* region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E. coli* and in Agrobacteria. They comprise a selection marker gene and a linker or polylinker which are framed by the right and left T-DNA border regions. They can be transformed directly into Agrobacteria (Holsters *et al.* [1978] *Mol. Gen. Genet.* 163:181-187). The *Agrobacterium* used as host cell is to comprise a plasmid carrying a *vir* region. The *vir* region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

## Claims

1. A method for controlling a cutworm pest wherein said method comprises contacting said pest with a *Cry*1F toxin.

2. The method of claim 1 wherein said toxin is a *Cry*1Fa toxin.

3. The method of claim 1 wherein said toxin is a truncated toxin.

4. The method of claim 1 wherein said toxin is in a plant cell that produced said toxin and wherein said pest contacts said toxin by ingesting said plant cell.

**5.** The method of claim 1 wherein said cutworm pest is of the genus *Agrotis*.

**6.** The method of claim 1 wherein said cutworm pest is an *Agrotis ipsilon.*

**7.** The method of claim 1 wherein said cutworm pest is an *Agrotis malefida*.

**8.** The method of claim 1 wherein said cutworm pest is of the genus *Porosagrotis.*

**9.** The method of claim 1 wherein said cutworm pest is a *Porosagrotis gypaetiana*.

**10.** The method of claim 1 wherein said cutworm pest is of the genus *Xylomyges.*

**11.** The method of claim 1 wherein said cutworm pest is a *Xylomyges curialis.*

**12.** The method of claim 1 wherein said cutworm pest is of the Tribe Agrotini

**13.** The method of claim 1 wherein said cutworm pest is of the genus *Feltia.*

**14.** The method of claim 1 wherein said cutworm pest is a *Feltia jaculifera*.

**15.** The method of claim 1 wherein said cutworm pest is of the genus *Euxoa*.

**16.** The method of claim 1 wherein said cutworm pest is an *Euxoa messoria*.

**17.** The method of claim 1 wherein said cutworm pest is an *Euxoa scandens*.

**18.** The method of claim 1 wherein said cutworm pest is an *Euxoa auxiliaris*.

**19.** The method of claim 1 wherein said cutworm pest is an *Euxoa detersa*.

**20.** The method of claim 1 wherein said cutworm pest is an *Euxoa tessellata*.

**21.** The method of claim 1 wherein said cutworm pest is an *Euxoa ochrogaster.*

**22.** The method of claim 1 wherein said cutworm pest is of the genus *Peridroma.*

**23.** The method of claim 1 wherein said cutworm pest is *a Peridroma saucia*.

**24.** The method of claim 4 wherein said plant is a corn plant.

**25.** The method of claim 4 wherein said plant is a sunflower plant.

**26.** The method of claim 4 wherein said plant is a soybean plant.

**27.** The method of claim 4 wherein said plant is a canola plant.

**28.** The method of claim 4 wherein said plant is a cotton plant.

**29.** The method of claim 4 wherein said contacting step is preceded by the step of planting a seed for a plant that comprises a polynucleotide that encodes said toxin.

**Patentansprüche**

**1.** Verfahren zur Bekämpfung eines Eulenfalterraupen ("Cutworm")-Schädlings, wobei das Verfahren das Kontaktieren des Schädlings mit einem *Cry*1F-Toxin umfasst.

**2.** Verfahren nach Anspruch 1, worin das Toxin ein *Cry*1Fa-Toxin ist.

3. Verfahren nach Anspruch 1, worin das Toxin ein verkürztes Toxin ist.

4. Verfahren nach Anspruch 1, worin das Toxin in einer Pflanzenzelle vorliegt, welche das Toxin produzierte, und worin der Schädling durch Aufnahme der Pflanzenzelle in Kontakt mit dem Toxin kommt.

5. Verfahren nach Anspruch 1, worin der Eulenfalterraupen-Schädling aus der Gattung *Agrotis* ist.

6. Verfahren nach Anspruch 1, worin der Eulenfalterraupen-Schädling ein *Agrotis ipsilon* ist.

7. Verfahren nach Anspruch 1, worin der Eulenfalterraupen-Schädling ein *Agrotis malefida* ist.

8. Verfahren nach Anspruch 1, worin der Eulenfalterraupen-Schädling aus der Gattung *Porosagrotis* ist.

9. Verfahren nach Anspruch 1, worin der Eulenfalterraupen-Schädling ein *Porosagrotis gypaetiana* ist.

10. Verfahren nach Anspruch 1, worin der Eulenfalterraupen-Schädling aus der Gattung *Xylomyges* ist.

11. Verfahren nach Anspruch 1, worin der Eulenfalterraupen-Schädling ein *Xylomyges curialis* ist.

12. Verfahren nach Anspruch 1, worin der Eulenfalterraupen-Schädling aus dem Stamm Agrotini ist.

13. Verfahren nach Anspruch 1, worin der Eulenfalterraupen-Schädling aus der Gattung *Feltia* ist.

14. Verfahren nach Anspruch 1, worin der Eulenfalterraupen-Schädling ein *Feltia jaculifera* ist.

15. Verfahren nach Anspruch 1, worin der Eulenfalterraupen-Schädling aus der Gattung *Euxoa* ist.

16. Verfahren nach Anspruch 1, worin der Eulenfalterraupen-Schädling ein *Euxoa messoria* ist.

17. Verfahren nach Anspruch 1, worin der Eulenfaiterraupen-Schädling ein *Euxoa scandens* ist.

18. Verfahren nach Anspruch 1, worin der Eulenfalterraupen-Schädling ein *Euxoa auxiliaris* ist.

19. Verfahren nach Anspruch 1, worin der Eulenfalterraupen-Schädling ein *Euxoa detersa* ist.

20. Verfahren nach Anspruch 1, worin der Eulenfalterraupen-Schädling ein *Euxoa tessellata* ist.

21. Verfahren nach Anspruch 1, worin der Eulenfalterraupen-Schädling ein *Euxoa ochrogaster* ist.

22. Verfahren nach Anspruch 1, worin der Eulenfalterraupen-Schädling aus der Gattung *Peridroma* ist.

23. Verfahren nach Anspruch 1, worin der Eulenfalterraupen-Schädling ein *Peridroma saucia* ist.

24. Verfahren nach Anspruch 4, worin die Pflanze eine Getreidepflanze ist.

25. Verfahren nach Anspruch 4, worin die Pflanze eine Sonnenblumenpflanze ist.

26. Verfahren nach Anspruch 4, worin die Pflanze eine Sojabohnenpflanze ist.

27. Verfahren nach Anspruch 4, worin die Pflanze eine Canolapflanze ist.

28. Verfahren nach Anspruch 4, worin die Pflanze eine Baumwollpflanze ist.

29. Verfahren nach Anspruch 4, worin dem Kontaktierungsschritt der Schritt des Pflanzens eines Samens für eine Pflanze vorausgeht, die ein Polynukleotid umfasst, welches für das Toxin kodiert.

## Revendications

1. Procédé de lutte contre un nuisible ver gris où ledit procédé comprend la mise en contact dudit nuisible avec une toxine *Cry*1F.

2. Procédé selon la revendication 1 où ladite toxine est une toxine *Cry*1Fa.

3. Procédé selon la revendication 1 où ladite toxine est une toxine tronquée.

4. Procédé selon la revendication 1 où ladite toxine est dans une cellule végétale qui a produit ladite toxine et où ledit nuisible vient en contact avec ladite toxine en ingérant ladite cellule végétale.

5. Procédé selon la revendication 1 où ledit nuisible ver gris est du genre *Agrotis*.

6. Procédé selon la revendication 1 où ledit nuisible ver gris est un *Agrotis ipsilon*.

7. Procédé selon la revendication 1 où ledit nuisible ver gris est un *Agrotis malefida*.

8. Procédé selon la revendication 1 où ledit nuisible ver gris est du genre *Porosagrotis*.

9. Procédé selon la revendication 1 où ledit nuisible ver gris est un *Porosagrotis gypaetiana*.

10. Procédé selon la revendication 1 où ledit nuisible ver gris est du genre *Xylomyges*.

11. Procédé selon la revendication 1 où ledit nuisible ver gris est un *Xylomyges curialis*.

12. Procédé selon la revendication 1 où ledit nuisible ver gris est de la tribu Agrotini.

13. Procédé selon la revendication 1 où ledit nuisible ver gris est du genre *Feltia*.

14. Procédé selon la revendication 1 où ledit nuisible ver gris est un *Feltia jaculifera*.

15. Procédé selon la revendication 1 où ledit nuisible ver gris est du genre *Euxoa*.

16. Procédé selon la revendication 1 où ledit nuisible ver gris est un *Euxoa messoria*.

17. Procédé selon la revendication 1 où ledit nuisible ver gris est un *Euxoa scandens*.

18. Procédé selon la revendication 1 où ledit nuisible ver gris est un *Euxoa auxiliaris*.

19. Procédé selon la revendication 1 où ledit nuisible ver gris est un *Euxoa detersa*.

20. Procédé selon la revendication 1 où ledit nuisible ver gris *est* un *Euxoa tessellata*.

21. Procédé selon la revendication 1 où ledit nuisible ver gris est un *Euxoa ochrogaster*.

22. Procédé selon la revendication 1 où ledit nuisible ver gris est du genre *Peridroma*.

23. Procédé selon la revendication 1 où ledit nuisible ver gris est un *Peridroma saucia*.

24. Procédé selon la revendication 4 où ladite plante est une plante de type maïs.

25. Procédé selon la revendication 4 où ladite plante est une plante de type tournesol.

26. Procédé selon la revendication 4 où ladite plante est une plante de type soja.

27. Procédé selon la revendication 4 où ladite plante est une plante de type colza.

**28.** Procédé selon la revendication 4 où ladite plante est une plante de type coton.

**29.** Procédé selon la revendication 4 où ladite étape de mise en contact est précédée par l'étape de plantation d'une graine pour une plante qui comprend un polynucléotide qui code ladite toxine.

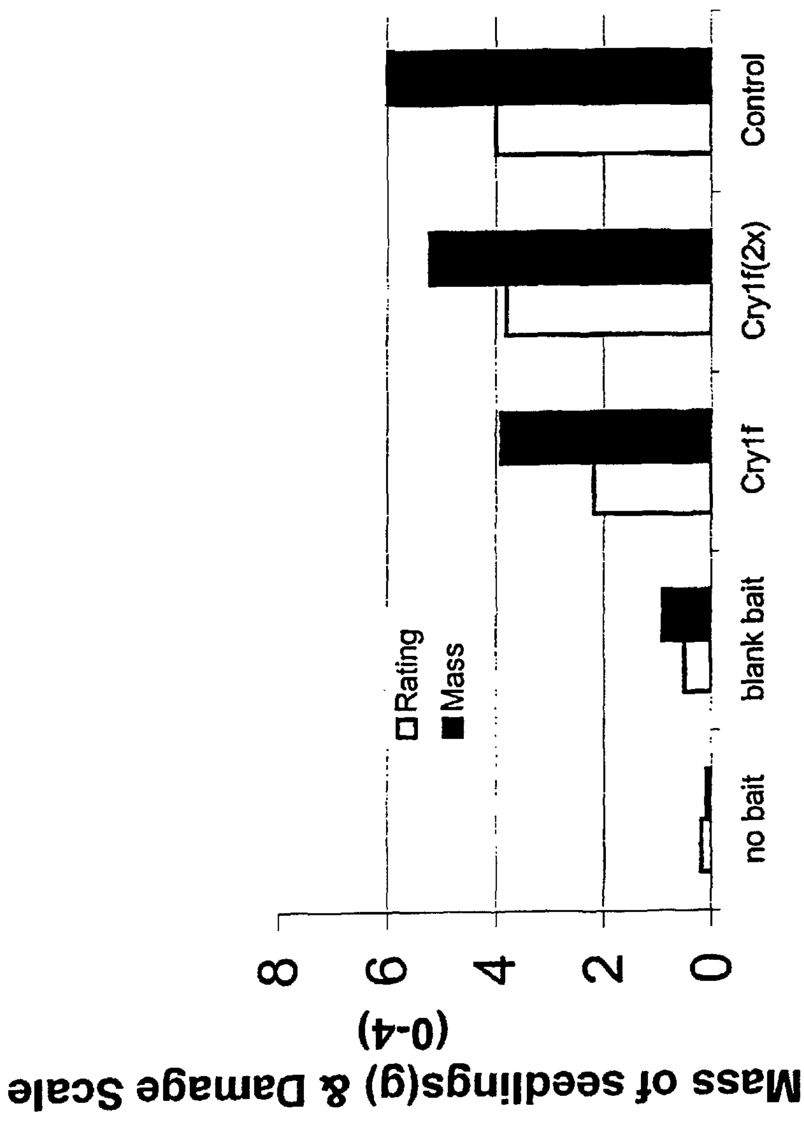
Mass of seedlings(g) & Damage Scale (0-4)
8
6
4
2
0
Rating
Mass
no bait
blank bait
Cry1f
Cry1f(2x)
Control

FIG. 1

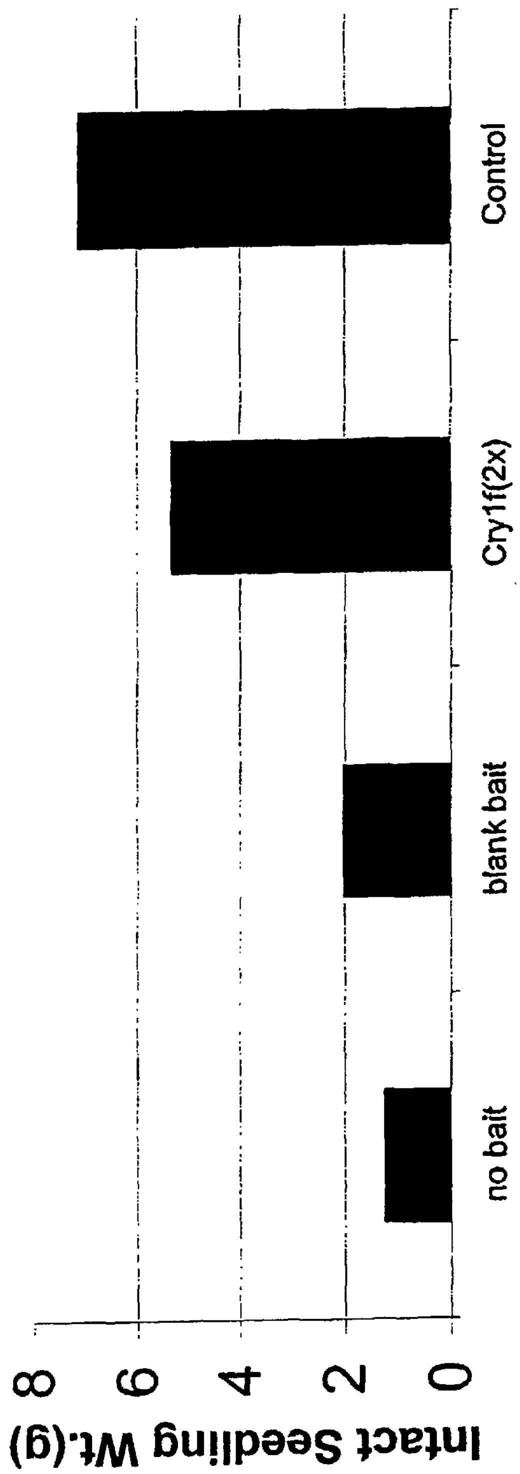
Intact Seedling Wt.(g)
0
2
4
6
8
no bait
blank bait
Cry1f(2x)
Control

FIG. 2

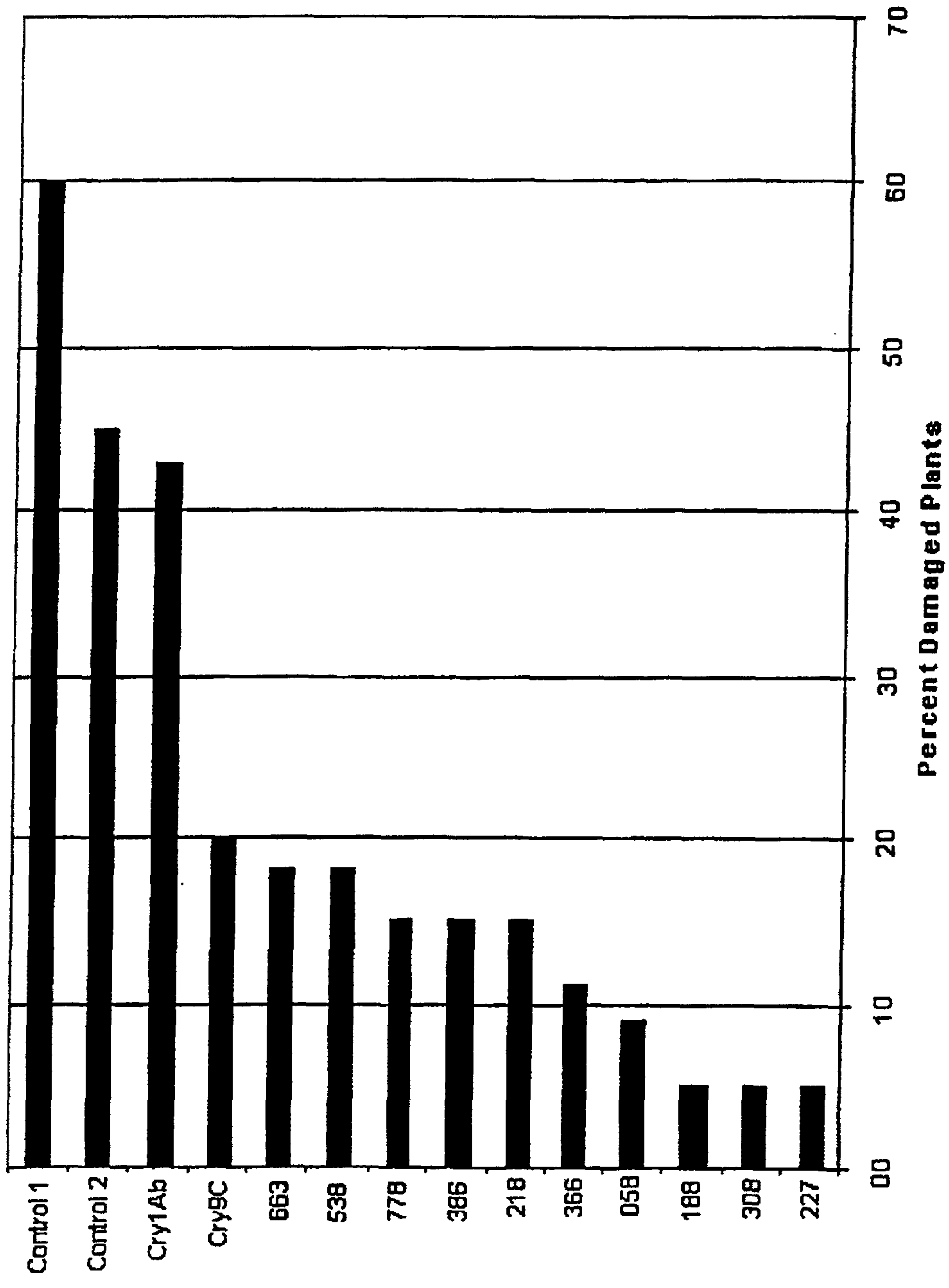
Control 1
Control 2
Cry1Ab
Cry9C
663
538
778
386
218
366
058
188
308
227
00
10
20
30
40
50
60
70
Percent Damaged Plants

FIG. 3

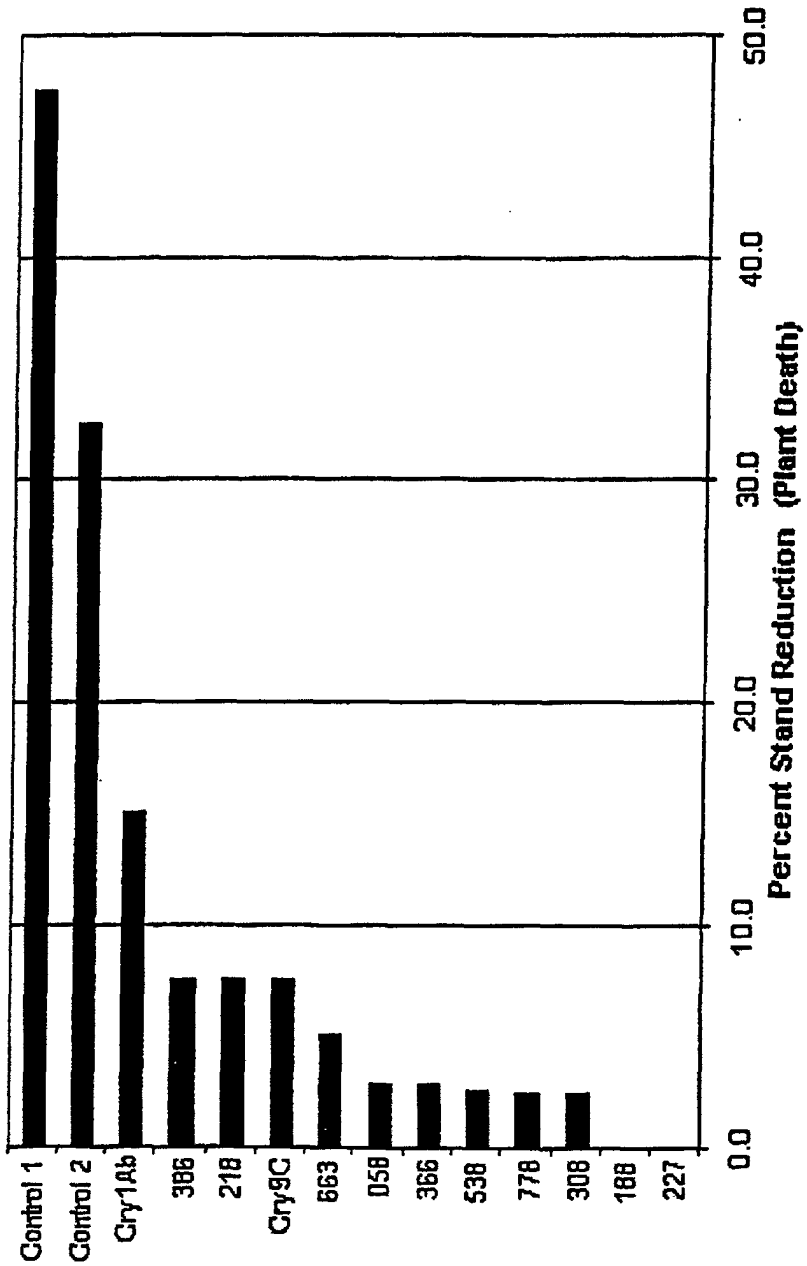
Control 1
Control 2
Cry1Ab
386
218
Cry9C
663
058
366
538
778
308
188
227
0.0
10.0
20.0
30.0
40.0
50.0
Percent Stand Reduction (Plant Death)

FIG. 4